(19) Europäisches Patentamt
European Patent Office
Office européen des brevets

(11) **EP 3 563 152 B1**

(12) **FASCICULE DE BREVET EUROPEEN**

(45) Date de publication et mention de la délivrance du brevet:
**16.02.2022   Bulletin 2022/07**

(21) Numéro de dépôt: **17825908.1**

(22) Date de dépôt: **15.12.2017**

(51) Classification Internationale des Brevets (IPC):
***G01N 33/22*** *(2006.01)*

(52) Classification Coopérative des Brevets (CPC):
**G01N 33/225**

(86) Numéro de dépôt international:
**PCT/FR2017/053613**

(87) Numéro de publication internationale:
**WO 2018/122490 (05.07.2018 Gazette 2018/27)**

(54) **PROCEDE D'ESTIMATION D'UNE CARACTERISTIQUE DE COMBUSTION D'UN GAZ CONTENANT DU DIHYDROGENE**

VERFAHREN ZUR SCHÄTZUNG EINER VERBRENNUNGSEIGENSCHAFT EINES WASSERSTOFFHALTIGEN GASES

METHOD FOR ESTIMATING A COMBUSTION CHARACTERISTIC OF A GAS CONTAINING HYDROGEN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **28.12.2016   FR 1663468**

(43) Date de publication de la demande:
**06.11.2019   Bulletin 2019/45**

(73) Titulaire: **Engie**
**92400 Courbevoie (FR)**

(72) Inventeurs:
• **OURLIAC, Mathieu**
**77165 Saint Soupplets (FR)**

• **CAPELA, Sandra**
**92210 SAINT-CLOUD (FR)**
• **LANTOINE, Laurent**
**78700 Conflans Sainte Honorine (FR)**
• **MANJOO, Naushad**
**95600 Eaubonne (FR)**

(74) Mandataire: **Cabinet Beau de Loménie**
**158, rue de l'Université**
**75340 Paris Cedex 07 (FR)**

(56) Documents cités:
**DE-B3-102008 029 553     US-A- 5 122 746**

**Description**

Arrière-plan de l'invention

L'invention se rapporte au domaine général de l'estimation de caractéristiques de combustion d'un gaz combustible appartenant à une famille de gaz combustibles, ladite au moins une caractéristique étant l'indice de Wobbe ou le pouvoir calorifique supérieur.

L'invention trouve notamment application dans l'estimation de caractéristiques de combustion d'un gaz combustible contenant du dihydrogène, par exemple un gaz combustible contenant une quantité de dihydrogène comprise entre 0% et 20% en volume.

L'utilisation de sources d'énergie renouvelables requiert parfois l'utilisation de moyens pour stocker de l'énergie car ces sources peuvent avoir un fonctionnement intermittent et décorrélé des besoins en énergie. Tel est notamment le cas lors de la production d'électricité photovoltaïque ou par éolienne.

Il a été proposé d'utiliser le dihydrogène obtenu par électrolyse de l'eau comme moyen de stockage de l'énergie provenant de sources d'énergie renouvelables.

Le dihydrogène ainsi généré peut ensuite être stocké dans des réservoirs, par exemple sous-terrain, ou il peut encore être injecté dans un réseau de distribution de gaz naturel. Le gaz obtenu peut ainsi comprendre entre 0% et 20% de dihydrogène.

L'indice de Wobbe (en kWh/Nm$^3$), qui est le rapport du pouvoir calorifique d'un gaz combustible sur la racine carrée de sa densité, et le pouvoir calorifique supérieur (en kWh/Nm$^3$) sont les deux principales grandeurs caractéristiques du combustible, qui influent sur les réglages de combustion des brûleurs à gaz - domestiques ou industriels.

Suivant le type d'application pour le gaz, et les organes de mesures mis en jeu (mesure de débit par organe déprimogène ou non), c'est l'un ou l'autre de ces deux paramètres qui est à considérer.

Par exemple, pour un injecteur à pression d'alimentation fixée (par exemple une table de cuisson, ou un brûleur rampe), la puissance délivrée dépend de la pression, de la section de passage de l'injecteur, de la perte de charge et de l'indice de Wobbe du combustible.

Pour des applications dans lesquelles on est muni d'un débit mètre massique ou volumique, la puissance délivrée dépend de la mesure du débit et du pouvoir calorifique.

En fait, le débit calorifique d'un brûleur est proportionnel soit à l'indice de Wobbe, soit au pouvoir calorifique supérieur (PCS). De la même façon, pour les gaz naturels distribués en Europe et sans hydrogène, la quantité d'air nécessaire pour obtenir une combustion stœchiométrique dépend de l'une ou de l'autre de ces caractéristiques du gaz combustible.

On notera que le réseau européen de transport et de distribution du gaz naturel est de plus en plus maillé et alimenté par des sources d'approvisionnement diverses, les caractéristiques du gaz naturel (Indice de Wobbe et PCS entre autres) peuvent donc varier de façon non négligeable (+/- 5% voire davantage) dans le temps, sur un point donné de ce réseau.

Cela étant, des procédés industriels tels que ceux mis en œuvre dans les industries du verre, de la céramique, de la production d'électricité, et de la chaux, de la métallurgie sont sensibles à ces variations. De ce fait, il est nécessaire, pour optimiser la combustion, de mettre en œuvre des solutions de régulation de combustion spécifiques. Pour mettre en œuvre une régulation de ce type, on peut mesurer l'indice de Wobbe ou du pouvoir calorifique supérieur.

Des solutions technologiques simples, robustes, et précises pour la mesure de ces paramètres, existent pour le gaz naturel. Cependant celles-ci sont déficientes lorsque du dihydrogène est présent dans le combustible. L'erreur dans la mesure de ces paramètres augmente fortement dès que l'on dépasse le pourcent en volume de dihydrogène, et la répétabilité des mesures n'est pas assurée.

De plus, des équipements industriels voire domestiques peuvent être sensibles à l'ajout de dihydrogène, même dans des proportions très faibles.

De ce fait, il existe un besoin de nouveaux appareils de mesures capables de quantifier, de manière continue, les paramètres de combustion majeurs de ces nouveaux combustibles : à la fois pour les usages sensibles tels que les procédés verriers ou métallurgiques, mais également pour que les opérateurs de réseaux puissent gérer localement les caractéristiques de combustion des combustibles gazeux fournis aux utilisateurs.

Des procédés ont été proposés pour mesurer ou estimer l'indice de Wobbe et le pouvoir calorifique.

Quatre familles d'appareils peuvent notamment être utilisées pour mesurer l'indice de Wobbe et/ou le pouvoir calorifique du gaz naturel, éventuellement avec une proportion non-nulle de dihydrogène :

- Le Calorimètre (pour le pouvoir calorifique) : La mesure implique la combustion d'une quantité contrôlée de gaz. L'énergie dégagée est ensuite quantifiée par écart de température entrée/sortie au niveau d'un échangeur (gaz ou eau). La précision est de l'ordre de 10% (soit 1,1 kWh/Nm$^3$) mais chaque mesure prend environ 10 minutes. Cette technologie est peu à peu abandonnée.

- Le Comburimètre (pour l'indice de Wobbe) : la mesure implique la combustion d'un mélange air/gaz. Le taux d'oxygène dans les produits de combustion est mesuré par une sonde zircone. Or, le taux d'oxygène résiduel est corrélé à l'indice de comburité, luimême corrélé, pour les gaz naturels, à l'indice de Wobbe. Le coût de ce type d'appareil est de l'ordre de 15000€. Ce type d'équipement fonctionne pour des mélanges comprenant une faible teneur en dihydrogène. (pour des taux de dihydrogène supérieurs à 5% en volume, l'indice de Wobbe n'est plus linéairement proportionnel à l'indice de comburité). L'inconvénient de cette technologie est la faible précision, de l'ordre de 5% (soit 0.75 kWh/Nm3) et la maintenance importante (vieillissement dues aux températures élevées du four dans lequel la combustion du mélange est réalisés).

- Le chromatographe en phase gazeuse (pour l'indice de Wobbe et pour le pouvoir calorifique) : Cette technique permet de séparer des molécules d'un mélange de gaz. L'indice de Wobbe et le pouvoir calorifique peuvent alors être calculés sur la base de la connaissance de la composition du gaz. Pour les équipements thermiques de forte puissance (turbines à gaz par exemple), les chromatographes en phase gazeuse ont généralement remplacé les calorimètres et comburimètres. La précision de ces appareils est meilleure, de l'ordre de 0, 5% (soit 0.08 kWh/Nm3 sur l'indice de Wobbe). Ce type d'équipement peut fonctionner aussi pour des mélanges de gaz naturel et de dihydrogène à la condition qu'il soit équipé d'un capteur spécifique permettant la séparation de la molécule de dihydrogène. L'inconvénient majeur des chromatographes en phase gazeuse est leur prix, même compte tenu des améliorations récentes (le prix peut être compris entre 20000€ et 50000€). En outre, le temps de réponse de ces chromatographes en phase gazeuse est de l'ordre de la minute dans le meilleur des cas. Il peut donc y avoir un décalage entre le gaz mesuré et celui réellement utilisé par l'équipement thermique (par exemple un brûleur).

- Les appareils à corrélation (pour l'indice de Wobbe ou pour le pouvoir calorifique supérieur) : Une(des) grandeur(s) physique(s), corrélée(s) à l'indice de Wobbe ou au pouvoir calorifique supérieur est(sont) mesurée(s) dans ces appareils. Une corrélation, implémentée par un calculateur, est alors utilisée pour estimer l'indice de Wobbe ou le PCS. Ce type d'appareil permet d'avoir une précision jusqu'à 1% pour les gaz naturel tels que ceux distribués en Europe. Les avantages de ce type de technologie sont la rapidité de la réponse (instantanée), le coût (entre 10000€ et 20000€), la robustesse et la maintenance réduite. Cependant, les appareils commercialisés ne fonctionnement pas pour des mélange gaz naturel et dihydrogène. Dès quelques pourcents de dihydrogène en volume dans un gaz naturel réel (non composé de méthane pur), l'erreur augmente drastiquement.

De l'état de la technique antérieure, on connaît le document EP 1 346 215 qui décrit un appareil de mesure de l'indice de Wobbe par corrélation.

On connaît également le document US 4 384 792 qui décrit un appareil de mesure et de régulation de l'indice de Wobbe d'un combustible gazeux par corrélations, et une régulation de l'indice de Wobbe.

On connaît également le document US 6 244 097 qui divulgue un appareil de mesure du pouvoir calorifique d'un combustible gazeux par corrélations.

On connaît le document DE 4 118 781 qui décrit un appareil de mesure du pouvoir calorifique et de l'indice de Wobbe d'un combustible gazeux par corrélations.

Enfin, on connaît le document US 5,122,746 qui décrit un appareil de mesure du pouvoir calorifique d'un combustible gazeux à base de mesures de la pression, de la température et de la composition. Z

Les solutions présentées dans ces documents présentent les inconvénients des appareils à corrélation. En particulier, ils ne sont pas utilisables dès lors que le gaz combustible étudié comporte du dihydrogène car leur précision est trop faible.

En outre, et comme indiqué ci-avant, les autres appareils qui peuvent fonctionner avec des mélanges de gaz naturel et de dihydrogène sont particulièrement coûteux, ce qui limite leurs applications aux seuls équipements de fortes puissances qui peuvent être installées chez les grand groupes industriels.

L'invention vise notamment à pallier certains au moins de ces inconvénients.

Objet et résumé de l'invention

La présente invention répond à ce besoin en proposant un procédé d'estimation d'au moins une caractéristique de combustion d'un gaz combustible appartenant à une famille de gaz combustibles, ladite au moins une caractéristique étant l'indice de Wobbe ou le pouvoir calorifique supérieur, le procédé comprenant :

une mesure d'au moins deux propriétés d'écoulement dudit gaz combustible,
une mesure du taux $X_{H_2}$ de dihydrogène contenu dans ledit gaz combustible,
ladite au moins une caractéristique $\Xi_{GN/H2}$ étant estimée au moyen de la loi affine empirique suivante :

$$\Xi_{GN/H2} = \alpha + \beta \cdot Y + \gamma \cdot X_{H_2}$$

avec $\alpha$, $\beta$, et $\gamma$ des coefficients prédéterminés pour la famille de gaz combustibles, et

Y une variable représentative de propriétés physiques dudit gaz combustible élaborée à partir desdites valeurs mesurées desdites au moins deux propriétés d'écoulement dudit gaz combustible.

Ce procédé concerne donc une méthode du type par corrélation, et ce procédé peut donc être mise en œuvre de manière simple et peu coûteuse, et ce procédé permet d'obtenir des résultats très rapides.

L'invention tient compte du taux de dihydrogène contenu dans le gaz combustible alors que tel n'est pas le cas des méthodes selon la technique antérieure.

On peut noter que l'invention trouve application pour des gaz combustible contenant entre 0% et 20% en volume de dihydrogène.

Les inventeurs ont observé qu'il était possible d'utiliser une loi affine empirique de même forme pour estimer à la fois le pouvoir calorifique supérieur et l'indice de Wobbe.

A titre indicatif, des propriétés d'écoulement différentes du gaz combustible peuvent être mesurées, selon qu'un pouvoir calorifique supérieur ou qu'un indice de Wobbe soit mesuré. En d'autre terme, la variable Y peut avoir une forme différente selon qu'un pouvoir calorifique supérieur ou qu'un indice de Wobbe soit mesuré.

Aussi, les coefficients $\alpha$, $\beta$, et $\gamma$ peuvent ne pas être fixes et être différents selon qu'un pouvoir calorifique supérieur ou qu'un indice de Wobbe soit mesuré.

On notera par ailleurs que les propriétés d'écoulement du gaz combustible sont des propriétés mesurées dans un organe d'écoulement et qui peuvent être choisis dans le groupe comprenant le débit, la température, ou encore la pression.

La variable Y représente des propriétés physiques du gaz. A titre indicatif, la variable Y peut être associée à une ou plusieurs propriétés physiques telles que la viscosité du gaz combustible, la capacité calorifique massique, ou encore la densité. Par être représentative de, ou être associée à, on entend que la variable Y peut-être écrite sous la forme d'une fonction mathématique liant ces propriétés physiques. La variable Y peut en fait être écrite à la fois sous la forme d'une fonction mathématique liant les propriétés physiques, mais également sous la forme d'une fonction mathématique liant les propriétés d'écoulement du gaz combustible car celles-ci sont utilisées pour l'élaboration de la variable Y. En effet, pour certains types de mesures de propriétés d'écoulement, il est possible de lier plusieurs de ces mesures à des propriétés physiques du gaz combustible de l'écoulement.

Aussi, en notant $U_{mes,1}$ la mesure de la propriété d'écoulement $U_1$, $U_{mes,2}$ la mesure de la propriété d'écoulement $U_2$, et $U_{mes,3}$ la mesure de la propriété d'écoulement $U_3$, on a alors :

$$Y = f\left(U_{mes,1}; U_{mes,2}; U_{mes,3}\right)$$

Selon un mode particulier de mise en œuvre, les coefficients $\alpha$, $\beta$, et $\gamma$ sont des coefficients lus dans une cartographie ayant pour entrée la valeur mesurée du taux $X_{H2}$ de dihydrogène et délivrant en sortie lesdits coefficients $\alpha$, $\beta$, et $\gamma$.

Ceci permet d'obtenir une estimation encore plus précise de la caractéristique à estimer.

Selon un mode particulier de mise en œuvre, ladite cartographie associe à des gammes de valeurs larges de 1% de taux $X_{H2}$ de dihydrogène lesdits coefficients $\alpha$, $\beta$, et $\gamma$.

En d'autres termes, la cartographie a une granularité de l'ordre du pourcent.

Les inventeurs de la présente invention ont observé que cela permettait d'obtenir une précision satisfaisante et ce avec un niveau de complexité acceptable.

Selon un mode particulier de mise en œuvre, les valeurs desdits coefficients $\alpha$, $\beta$, et $\gamma$ sont obtenues à partir d'un ensemble de données relatives à des gaz connus de ladite famille de gaz combustibles pour lesquels on connaît les caractéristiques de combustion et la valeur de Y représentatives desdites propriétés physiques.

Par gaz connus, on entend des gaz dont on connaît la composition, par exemple des gaz dont on connaît les proportions des différents constituants de nature chimique déterminée. L'homme du métier saura déterminer les caractéristiques de combustion de ces gaz, et il pourra en particulier utiliser des gaz connus comprenant une quantité non-nulle de dihydrogène.

Selon un mode particulier de mise en œuvre, le procédé comprend une génération aléatoire de caractéristiques de combustion et de valeurs de Y représentatives de propriétés physiques à partir dudit ensemble de données relatives à des gaz connus de ladite famille de gaz combustibles.

Selon un mode particulier de mise en œuvre, on estime l'indice de Wobbe et le pouvoir calorifique supérieur au moyen de deux lois affines empiriques.

Dans une même mise en œuvre du procédé, on peut donc estimer ces deux caractéristiques de combustion.

Selon un mode particulier de mise en œuvre, le procédé comprend en outre une estimation de la densité dudit gaz combustible à partir de l'indice de Wobbe estimé et du pouvoir calorifique supérieur estimé.

Selon un mode particulier de mise en œuvre, le procédé comprend une régulation de la caractéristique de combustion du gaz combustible ou une régulation de la caractéristique de combustion d'un gaz combustible et d'un volume d'air stœchiométrique estimé ou d'un indice de comburité estimé correspondant à ladite caractéristique estimée.

Selon un mode particulier de mise en œuvre, ladite au moins une caractéristique de combustion du gaz combustible comprend l'indice de Wobbe $IW_{GN/H2}$,

ladite mesure d'au moins deux propriétés d'écoulement dudit gaz combustible comporte une mesure d'un débit massique du gaz combustible en écoulement sonique (c'est-à-dire à une vitesse supérieure ou égale à celle du son) à travers une restriction fluidique (par exemple un orifice ou une micro-tuyère), effectuée à une pression absolue mesurée en amont de la restriction et à une température absolue mesurée en amont de la restriction,

le procédé comprenant en outre une procédure d'étalonnage au cours de laquelle est effectuée une mesure d'un débit massique d'un gaz de référence (par exemple du méthane) en écoulement sonique à travers ladite restriction fluidique, à une pression absolue de référence mesurée en amont de la restriction et à une température absolue de référence mesurée en amont de la restriction ;

la loi affine empirique utilisée pour estimer l'indice de Wobbe $IW_{GN/H2}$ s'écrivant alors :

$$IW_{GN/H2} = D + E \cdot Y + F \cdot X_{H_2}$$

avec :

$$Y = \frac{Q_{mes,2}}{Q_{ref}} \cdot \frac{p_{ref}}{p_{mes}} \cdot \sqrt{\frac{T_{ref}}{T_{mes}}}$$

où $Q_{mes,2}$ est le débit massique du gaz combustible mesuré,

$p_{mes}$ est la pression absolue du gaz combustible mesurée,
$T_{mes}$ est la température absolue du gaz combustible mesurée,
$Q_{ref}$ est le débit massique du gaz de référence mesuré,
$p_{ref}$ est la pression absolue du gaz de référence mesurée, et
$T_{ref}$ est la température absolue du gaz de référence mesurée ; et D, E, et F sont des coefficients prédéterminés pour la famille de gaz combustibles et correspondant respectivement aux coefficients $\alpha$, $\beta$, et $\gamma$.

Selon un mode particulier de mise en œuvre, on a estimé l'indice de Wobbe dudit gaz combustible, et le procédé comprend en outre une mesure de la densité dudit gaz combustible et une estimation du pouvoir calorifique supérieur à partir de l'indice de Wobbe estimé et de la densité du gaz mesurée.

Selon un mode particulier de mise en oeuvre, ladite au moins une caractéristique de combustion du gaz combustible comprend le pouvoir calorifique supérieur $PCS_{GN/H2}$,
ladite mesure d'au moins deux propriétés d'écoulement dudit gaz combustible comporte :

une mesure de débit massique dudit gaz combustible en écoulement laminaire à travers un appareil faisant apparaître une chute de pression, la mesure dépendant de la viscosité du gaz combustible et de la viscosité d'un gaz de référence (par exemple du méthane), et

une mesure, en aval dudit appareil faisant apparaître une chute de pression, du débit massique dudit gaz combustible au moyen d'un débitmètre massique thermique, la mesure dépendant de la capacité thermique massique du gaz combustible et de la capacité thermique du gaz de référence ;

la loi affine empirique utilisée pour estimer le pouvoir calorifique supérieur $PCS_{GN/H2}$ s'écrivant alors

$$PCS_{GN/H2} = A + B \cdot Z + C \cdot X_{H_2}$$

avec :

$$Z = \frac{Q_{mes,1}}{Q_{mes,2}}$$

où z est une variable correspondant à la variable Y,

$Q_{mes,1}$ est le débit massique du gaz combustible en écoulement laminaire à travers un appareil faisant apparaître une chute de pression mesuré, et

$Q_{mes,2}$ est le débit massique du gaz combustible mesuré en aval dudit appareil faisant apparaître une chute de pression ; et A, B, et C sont des coefficients prédéterminés pour la famille de gaz combustibles et correspondant respectivement aux coefficients $\alpha$, $\beta$, et $\gamma$.

On pourra mettre en œuvre une étape d'étalonnage avec le gaz de référence, par exemple pour obtenir les valeurs de viscosité et de capacité thermique du gaz de référence.

Cette étape d'étalonnage permet d'obtenir des mesures justes, et en particulier pour les gaz qui ne sont pas purs tels que des gaz qui comportent du dihydrogène.

L'invention propose également un dispositif d'estimation d'au moins une caractéristique de combustion d'un gaz combustible appartenant à une famille de gaz combustibles, ladite au moins une caractéristique étant l'indice de Wobbe ou le pouvoir calorifique supérieur, le dispositif comprenant :

au moins deux modules de mesure d'au moins deux propriétés d'écoulement dudit gaz combustible,
un module de mesure du taux $X_{H2}$ de dihydrogène contenu dans ledit gaz combustible,
un module configuré pour estimer ladite au moins une caractéristique $\Xi_{GN/H2}$ au moyen de la loi affine empirique suivante :

$$\Xi_{GN/H2} = \alpha + \beta \cdot Y + \gamma \cdot X_{H_2}$$

avec $\alpha$, $\beta$, et $\gamma$ des coefficients prédéterminés pour la famille de gaz combustibles, et
Y une variable représentative de propriétés physiques dudit gaz combustible élaborée à partir desdites valeurs mesurées desdites au moins deux propriétés d'écoulement dudit gaz combustible.

Ce dispositif peut être configuré pour la mise en œuvre de tous les modes de mise en œuvre du procédé tel que décrit ci-avant.

On notera que ce dispositif peut comprendre l'organe d'écoulement dans lequel s'écoule le gaz combustible pour que ses propriétés d'écoulement soient mesurées.

Selon un mode particulier de réalisation, ladite au moins une caractéristique de combustion du gaz combustible comprend l'indice de Wobbe $IW_{GN/H2}$, le dispositif comprenant :

une entrée pour recevoir un flux dudit gaz combustible,
une entrée pour recevoir un flux d'un gaz de référence,
un module de sélection et de guidage pour amener le flux dudit gaz combustible ou le flux dudit gaz de référence à une conduite,
une restriction fluidique,
un module de mesure d'un débit massique du gaz combustible en écoulement sonique à travers ladite restriction fluidique, comprenant un sous-module de mesure de la pression absolue en amont de la restriction et un sous-module de mesure de la température absolue en amont de la restriction,
la loi affine empirique utilisée pour estimer l'indice de Wobbe $IW_{GN/H2}$ s'écrivant alors :

$$IW_{GN/H2} = D + E \cdot Y + F \cdot X_{H_2}$$

avec :

$$Y = \frac{Q_{mes,2}}{Q_{ref}} \cdot \frac{p_{ref}}{p_{mes}} \cdot \sqrt{\frac{T_{ref}}{T_{mes}}}$$

où $Q_{mes,2}$ est le débit massique du gaz combustible mesuré,

$p_{mes}$ est la pression absolue du gaz combustible mesurée,

$T_{mes}$ est la température absolue du gaz combustible mesurée,

$Q_{ref}$ est le débit massique du gaz de référence mesuré,

$p_{ref}$ est la pression absolue du gaz de référence mesurée, et

$T_{ref}$ est la température absolue du gaz de référence mesurée ; et D, E, et F sont des coefficients prédéterminés pour la famille de gaz combustibles et correspondant respectivement aux coefficients $\alpha$, $\beta$, et $\gamma$.

Selon un mode particulier de réalisation, le dispositif est apte à estimer l'indice de Wobbe dudit gaz combustible, le dispositif comprenant en outre un module de mesure de la densité dudit gaz combustible et le module configuré pour estimer ladite au moins une caractéristique étant en outre configuré pour estimer le pouvoir calorifique supérieur à partir de l'indice de Wobbe estimé et de la densité du gaz mesurée.

On pourra notamment obtenir le pouvoir calorifique supérieur en multipliant l'indice de Wobbe estimé par le carré de la densité mesurée.

A titre indicatif, on notera qu'il est possible d'utiliser un capteur de densité de la société suisse TRAFAG et portant la référence 8774.

Selon un mode particulier de réalisation, ladite au moins une caractéristique de combustion du gaz combustible comprend le pouvoir calorifique supérieur $PCS_{GN/H2}$, le dispositif comprenant :

une entrée pour recevoir un flux dudit gaz combustible,

un module de mesure de débit massique dudit gaz combustible en écoulement laminaire à travers un appareil faisant apparaître une chute de pression, la mesure dépendant de la viscosité du gaz combustible et de la viscosité d'un gaz de référence, et

un module de mesure, en aval dudit appareil faisant apparaître une chute de pression, du débit massique dudit gaz combustible au moyen d'un débitmètre massique thermique, la mesure dépendant de la capacité thermique massique du gaz combustible et de la capacité thermique du gaz de référence ;

la loi affine empirique utilisée pour estimer le pouvoir calorifique supérieur $PCS_{GN/H2}$ s'écrivant alors

$$PCS_{GN/H2} = A + B \cdot Z + C \cdot X_{H_2}$$

avec :

$$Z = \frac{Q_{mes,1}}{Q_{mes,2}}$$

où Z est une variable correspondant à la variable Y,

$Q_{mes,1}$ est le débit massique du gaz combustible en écoulement laminaire à travers un appareil faisant apparaître une chute de pression mesuré, et

$Q_{mes,2}$ est le débit massique du gaz combustible mesuré en aval dudit appareil faisant apparaître une chute de pression ; et A, B, et C sont des coefficients prédéterminés pour la famille de gaz combustibles et correspondant respectivement aux coefficients $\alpha$, $\beta$, et $\gamma$.

Selon un mode particulier de réalisation, lequel le module configuré pour estimer ladite au moins une caractéristique $\Xi_{GN/H2}$ au moyen de la loi affine empirique est en outre configuré pour estimer un volume d'air stœchiométrique ou un indice de comburité.

Selon un mode particulier de réalisation, le dispositif comprend en outre un module de régulation de ladite caractéristique de combustion du gaz combustible ou de régulation de la caractéristique de combustion d'un gaz combustible et d'un volume d'air stœchiométrique estimé ou d'un indice de comburité estimé correspondant à ladite caractéristique estimée.

Ce module de régulation peut notamment comprendre un actionneur pour injecter un gaz supplémentaire, par exemple de l'air. La régulation peut être en boucle fermée.

Brève description des dessins

D'autres caractéristiques et avantages de la présente invention ressortiront de la description faite ci-dessous, en référence aux dessins annexés qui en illustrent un exemple dépourvu de tout caractère limitatif.

Sur les figures :

- la figure 1 représente de façon schématique les étapes d'un exemple d'un procédé d'estimation d'une caractéristique de combustion,
- la figure 2 représente de façon schématique un exemple de dispositif d'estimation d'une caractéristique de combustion,
- la figure 3 représente de façon schématique les étapes d'un procédé d'estimation de l'indice de Wobbe,
- la figure 4 représente de façon schématique un exemple de dispositif d'estimation de l'indice de Wobbe,
- la figure 5 représente de façon schématique les étapes d'un procédé d'estimation du pouvoir calorifique supérieur,
- la figure 6 représente de façon schématique un exemple de dispositif d'estimation du pouvoir calorifique supérieur,
- la figure 7 représente de façon schématique un exemple de dispositif d'estimation de l'indice de Wobbe et du pouvoir calorifique supérieur, et
- la figure 8 représente de façon schématique un exemple de dispositif de régulation de l'indice de Wobbe.

Description détaillée

Nous allons maintenant décrire un procédé et un dispositif d'estimation d'une caractéristique de combustion d'un gaz combustible appartenant à une famille de gaz combustibles. La caractéristique peut être l'indice de Wobbe ou le pouvoir calorifique supérieur.

L'invention n'est nullement limitée à l'estimation d'une seule caractéristique et peut comprendre l'estimation à la fois de l'indice de Wobbe et du pouvoir calorifique supérieur.

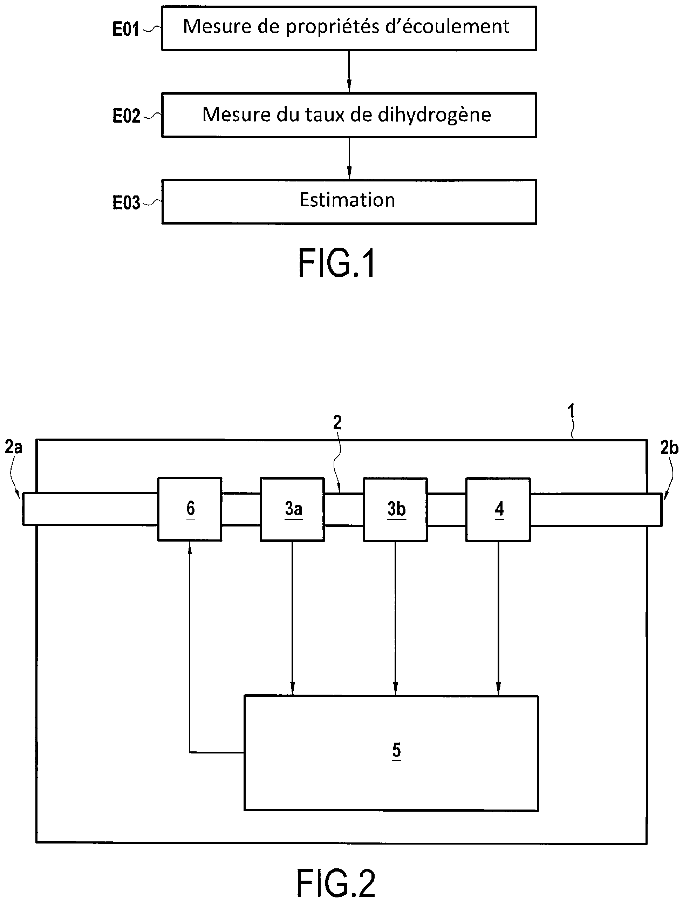

Sur la figure 1, on a représenté de manière schématique les étapes d'un procédé d'estimation d'une caractéristique de combustion d'un gaz combustible.

Ce procédé est particulièrement adapté aux gaz qui peuvent contenir une quantité non-nulle de dihydrogène, et en particulier aux gaz combustibles comprenant entre 0% et 20% de dihydrogène.

Dans une première étape du procédé E01, on mesure au moins deux propriétés d'écoulement du gaz combustible. Le gaz combustible d'étude est alors en écoulement dans un organe d'écoulement et on mesure, par exemple au moyens de capteurs, des propriétés d'écoulement telles que la température, la pression, ou encore le débit.

On mesure dans une deuxième étape E02 un taux de dihydrogène contenu dans le gaz en écoulement noté $X_{H2}$. On peut noter que dans tous les modes de mise en œuvre et de réalisation de l'invention, le taux de dihydrogène peut être une fraction molaire ou volumique. On peut considérer que si les pressions sont suffisamment faibles, la loi des gaz parfaits s'applique et les deux fractions molaire et volumique ont les mêmes valeurs. Dans des applications de l'invention, le taux de dihydrogène a la même valeur en fraction molaire et en fraction volumique. Cette étape peut être mise en œuvre simultanément à l'étape E01, ou préalablement, ou postérieurement.

Dans une troisième étape E03, on estime la caractéristique notée $\Xi_{GN/H2}$ au moyen de la loi affine empirique suivante :

$$\Xi_{GN/H2} = \alpha + \beta \cdot Y + \gamma \cdot X_{H_2}$$

avec $\alpha$, $\beta$, et $\gamma$ des coefficients prédéterminés pour la famille de gaz combustibles, et
Y une variable représentative de propriétés physiques dudit gaz combustible élaborée à partir desdites valeurs mesurées desdites au moins deux propriétés d'écoulement dudit gaz combustible.

Les valeurs mesurées desdites au moins deux propriétés d'écoulement dudit gaz combustible sont en fait choisies de sorte que la variable Y puisse être à la fois représentative de propriétés physiques du gaz combustible telles que la viscosité, la capacité calorifique massique, ou encore la densité, et exprimable en fonction des valeurs mesurées des propriétés d'écoulement du gaz combustible.

Aussi, en notant $U_{mes,1}$ la mesure de la propriété d'écoulement $U_1$, $U_{mes,2}$ la mesure de la propriété d'écoulement $U_2$, et
$U_{mes,3}$ la mesure de la propriété d'écoulement $U_3$, on a alors :

$$Y = f\left(U_{mes,1}; U_{mes,2}; U_{mes,3}\right)$$

La figure 2 est un exemple de réalisation d'un dispositif d'estimation d'au moins une caractéristique de combustion d'un gaz combustible appartenant à une famille de gaz combustibles.

Ce dispositif peut notamment mettre en œuvre un procédé tel que celui décrit en référence à la figure 1.

Ce dispositif 1 comprend ici un organe d'écoulement 2 configuré pour recevoir un écoulement d'un flux de gaz combustible pour lequel on souhaite estimer par exemple l'indice de Wobbe ou le pouvoir calorifique supérieur. L'organe d'écoulement comporte une entrée 2a pour recevoir le flux de gaz combustible et une sortie 2b.

Le dispositif 1 comporte en outre deux modules 3a et 3b de mesure de propriétés d'écoulement du gaz combustible.

Par exemple, les modules 3a et 3b peuvent chacun ou tous les deux mesurer un débit, une pression, ou encore une température.

Les modules 3a et 3b sont en fait des capteurs connus en tant que tels pour mesurer un débit, une pression, ou encore une température.

Le dispositif 1 comporte un module 4 de mesure d'un taux $X_{H_2}$ de dihydrogène, par exemple un capteur délivrant un pourcentage molaire de dihydrogène.

Les modules 3a, 3b et 4 sont en communication avec un module d'estimation 5, de manière à communiquer les résultats de leurs mesures respectives au module d'estimation 5. Le module d'estimation 5 peut être un calculateur muni d'un processeur et d'une mémoire (non représentés).

Le module 5 est configuré pour estimer ladite au moins une caractéristique $\Xi_{GN/H2}$ au moyen de la loi affine empirique suivante :

$$\Xi_{GN/H2} = \alpha + \beta \cdot Y + \gamma \cdot X_{H_2}$$

avec $\alpha$, $\beta$, et $\gamma$ des coefficients prédéterminés pour la famille de gaz combustibles, et

Y une variable représentative de propriétés physiques dudit gaz combustible élaborée à partir desdites valeurs mesurées par les modules 3a, 3b desdites au moins deux propriétés d'écoulement dudit gaz combustible.

A cet effet, le module 5 peut avoir, enregistré dans une mémoire, des valeurs possibles des coefficients prédéterminés $\alpha$, $\beta$, et $\gamma$. Le module 5 peut également avoir, enregistré dans une mémoire, des instructions de programme d'ordinateur provoquant l'exécution de l'estimation.

Par exemple, ce programme d'ordinateur peut comprendre une instruction de calcul de la variable Y à partir des valeurs mesurées par les modules 3a et 3b, et une instruction de calcul de la caractéristique $\Xi_{GN/H2}$ au moyen de la fonction définie ci-avant.

De manière optionnelle, le dispositif 1 peut comporter un actionneur 6 contrôlé par le module d'estimation 5 en vue de réguler au moins ladite caractéristique. L'actionneur 6 peut être par exemple un injecteur d'air comprimé.

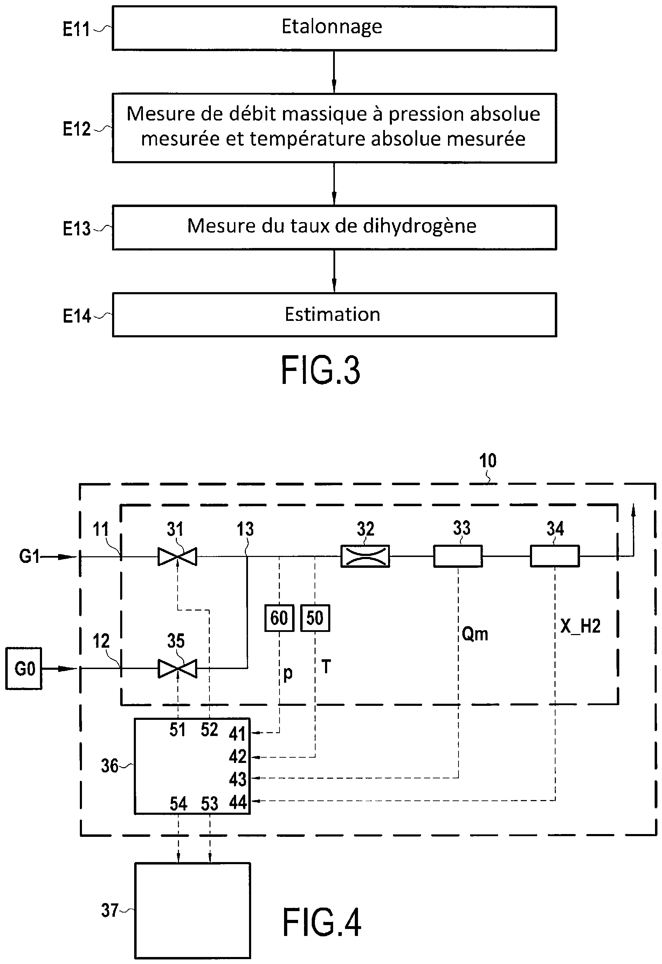

On va maintenant décrire, en se référant aux figures 3 et 4, un mode de mise en œuvre et de réalisation dans lequel on estime l'indice de Wobbe $IW_{GN/H2}$ d'un gaz combustible.

Sur la figure 3, on a représenté les étapes d'un procédé d'estimation de l'indice de Wobbe $IW_{GN/H2}$ d'un gaz combustible.

Ce procédé comporte une première étape E11 d'étalonnage au cours de laquelle est effectuée une mesure d'un débit massique d'un gaz de référence (par exemple du méthane) en écoulement sonique à travers une restriction fluidique (par exemple un orifice ou une micro-tuyère), à une pression absolue de référence mesurée et à une température absolue de référence mesurée.

Dans une deuxième étape E12, on mesure un débit massique du gaz combustible en écoulement sonique à travers la restriction fluidique, cette mesure étant effectuée à une pression absolue mesurée en amont de la restriction et à une température absolue mesurée en amont de la restriction.

Dans une étape E13, on mesure le taux de dihydrogène $X_{H_2}$.

Les étapes E11 à E13 peuvent être mise en œuvre dans tout ordre possible. En particulier, les étape E12 et E13 peuvent être mises en œuvre simultanément.

Dans une étape E14 on estime l'indice de Wobbe $IW_{GN/H2}$ par une loi affine empirique s'écrivant alors :

$$IW_{GN/H2} = D + E \cdot Y + F \cdot X_{H_2}$$

avec :

$$Y = \frac{Q_{mes,2}}{Q_{ref}} \cdot \frac{p_{ref}}{p_{mes}} \cdot \sqrt{\frac{T_{ref}}{T_{mes}}}$$

où $Q_{mes,2}$ est le débit massique du gaz combustible mesuré,

$p_{mes}$ est la pression absolue du gaz combustible mesurée,
$T_{mes}$ est la température absolue du gaz combustible mesurée,

$Q_{ref}$ est le débit massique du gaz de référence mesuré,

$p_{ref}$ est la pression absolue du gaz de référence mesurée, et

$T_{ref}$ est la température absolue du gaz de référence mesurée ; et *D, E,* et *F* sont des coefficients prédéterminés pour la famille de gaz combustibles et correspondant respectivement aux coefficients $\alpha$, $\beta$, et $\gamma$.

Pour mettre en oeuvre les étapes E11 et E12, on utilise une restriction fluidique tel qu'un orifice ou une micro-tuyère dont on connaît la géométrie.

De part et d'autre de la restriction fluidique, on effectue les mesures suivantes : en amont de la restriction fluidique, on mesure les températures absolues et les pressions absolues, et en aval de la restriction fluidique on mesure le débit massique (par exemple au moyen d'un débitmètre massique thermique).

En fait, cela correspond à mesurer de deux manières différentes le débit volumique normal noté $Q_{vn}$ traversant le dispositif.

En effet, l'écoulement étant sonique, on a :

$$Q_{vn} = k \cdot p \sqrt{\frac{T}{d}}$$

Avec :

*k* une constante caractéristique de la géométrie de l'orifice,

*p* et *T* la pression absolue et la température absolue du gaz en amont de l'orifice, et

*d* la densité du gaz.

Aussi, en utilisant le débitmètre massique thermique, on a :

$$Q_{vn} = Q_{mes,2} \cdot C$$

Avec :

$Q_{vn,mes,2}$ la mesure réalisée par le débitmètre massique thermique obtenue à l'étape E12,

C un coefficient correcteur qui tient compte des différences de propriétés physiques du gaz (par exemple la capacité calorifique, la viscosité, la conductivité thermique) par rapport à celles de l'air.

Pour un gaz de composition connue, on a :

$$\frac{1}{C} = \sum \frac{X_i}{C_i}$$

Avec :

$X_i$ la fraction volumique du constituant i

$C_i$ le coefficient correcteur relatif au constituant i. Ce coefficient peut être lu dans une table associée au débitmètre massique thermique.

L'étape d'étalonnage E11 est une étape au cours de laquelle on alimente le dispositif avec un gaz de référence dont la composition est parfaitement connue (de préférence du méthane pur).

L'égalité des deux relations de débit avec les valeurs obtenues dans la procédure d'étalonnage de l'étape E11 peut donc s'écrire :

$$Q_{vn,ref} \cdot C_{ref} = k \cdot p_{ref} \sqrt{\frac{T_{ref}}{d_{ref}}}$$

Et, dans la procédure de mesure de l'étape E12 dans laquelle on effectue des mesures sur le gaz combustible :

$$Q_{mes,2} \cdot C = k \cdot p_{mes} \sqrt{\frac{T_{mes}}{d}}$$

On a également connaissance du taux d'hydrogène (par exemple en volume) $X_{H2}$ au moyen de la mesure de l'étape E14.

On peut noter que dans les équations ci-dessus, les deux seuls paramètres inconnus sont $C$ et la densité $d$. On peut ensuite éliminer le paramètre $k$ pour exprimer la variable $Y$, liée à $C$ et à la densité $d$.

En fait, on a :

$$Y = \frac{C_{ref}}{C} \cdot \sqrt{\frac{d_{ref}}{d}}$$

Et, Y est une variable représentative de propriétés physiques du gaz combustible. On peut réécrire Y à partir des valeurs mesurées. Ces valeurs mesurées ($Q_{mes,2}$, $p$, et $T$) peuvent être notées $U_{mes,1}$ la mesure de la propriété d'écoulement $U_1$, $U_{mes,2}$ la mesure de la propriété d'écoulement $U_2$, et $U_{mes,3}$ la mesure de la propriété d'écoulement $U_3$, on a alors :

$$Y = f\left(U_{mes,1}; U_{mes,2}; U_{mes,3}\right)$$

Où:

$U_{mes,1} = p_{mes}$,
$U_{mes,2} = Q_{mes,2}$, et
$U_{mes,3} = T_{mes}$

Enfin, on a :

$$Y = \frac{C_{ref}}{C} \cdot \sqrt{\frac{d_{ref}}{d}} = \frac{Q_{mes,2}}{Q_{ref}} \cdot \frac{p_{ref}}{p_{mes}} \sqrt{\frac{T_{ref}}{T_{mes}}}$$

Cette définition de la variable Y peut être utilisée dans une loi affine empirique ayant pour variable Y, et qui s'écrit de la forme suivante :

$$IW_{GN/H2} = D + E \cdot Y + F \cdot X_{H_2}$$

$D, E,$ et $F$ étant des coefficients prédéterminés pour la famille de gaz combustibles et correspondant respectivement aux coefficients $\alpha$, $\beta$, et $\gamma$ décrits en référence aux figures 1 et 2.

Les coefficients $D, E,$ et $F$ peuvent être obtenus à partir de composition de gaz connues, par exemple des compositions de gaz naturelles du réseau d'un pays ou d'une région. Par exemple, on peut utiliser des compositions connues de gaz à haut pouvoir calorifique distribué en Europe et bien connu de l'homme du métier.

A partir de ces compositions connues, on peut définir les valeurs limites sur la fraction molaire des différents composés. Par exemple, en notant $X_K$ la fraction molaire de l'espèce K dans un gaz, on peut avoir des compositions connues de ce type :

$0\% < X_{N2} < 5.5\%$
$0.5 < X_{C2H6} < 12.5\%$
$0\% < X_{C4H10} < 3.5\%$
$0\% < X_{CO2} < 1.3\%$

$0\% < X_{C3H8} < 3.5\%$

$0\% < X_{C5H12} < 3.5\%$

$$X_{CH4} = 1 - \Sigma X\_k$$

On notera qu'il n'y a pas de dihydrogène dans les compositions actuelles de gaz.

Ces gammes possibles permettent de générer aléatoirement des compositions de gaz pour lesquelles on peut déterminer l'indice de Wobbe (ou même le pouvoir calorifique supérieur). 10000 gaz peuvent ainsi être générés.

On peut, pour chaque gaz généré aléatoirement, ajouter une quantité aléatoire de dihydrogène comprise entre 0% et 20% de fraction molaire. Ici encore, on peut déterminer l'indice de Wobbe de ces gaz générés aléatoirement.

On peut également déduire des compositions générées aléatoirement les valeurs associées de la variable Y. En appliquant une méthode des moindres carrés on peut obtenir des valeurs pour les coefficients *D, E,* et *F* :

$$IW_{GN/H2} = -18{,}0272 + 32{,}85887 \cdot Y - 0{,}11633 \cdot X_{H_2}$$

Ces résultats sont obtenus en utilisant le méthane comme gaz de référence.

On peut noter que pour une fraction molaire de dihydrogène comprise entre 0% et 20%, l'erreur dans l'estimation de l'indice de Wobbe est toujours inférieure à 1,4%, et qu'elle est inférieure à 1% pour 98% des 10 000 gaz obtenus aléatoirement.

De manière alternative, on peut utiliser une corrélation dans laquelle les coefficients ne sont pas des constantes et dépendent du taux de dihydrogène en fraction molaire.

On peut réécrire la loi affine empirique de la manière suivante :

$$IW_{GN/H2} = D(X_{H_2}) + E(X_{H_2}) \cdot Y$$

Ici, D et E sont des fonctions du taux de dihydrogène (l'équation a été réécrite pour ne faire apparaître que deux coefficients qui dépendent de $X_{H2}$, toutefois, il est possible d'écrire l'équation avec trois coefficients).

De la même manière, en générant des compositions aléatoires de gaz, les inventeurs ont observé qu'il est possible d'utiliser des fonctions D et E constantes par morceau sur des intervalles de concentration en dihydrogène de largeur 1%.

Par exemple, entre 2% et 3% de dihydrogène, *D* et *E* prennent les valeurs suivantes :

*D* = -18.181

*E* = 32.715

Il a été observé que l'erreur dans l'estimation de l'indice de Wobbe est alors toujours inférieure à 1% pour 95% des 10 000 gaz aléatoires.

Sur la figure 4, on a représenté un dispositif d'estimation de l'indice de Wobbe, pouvant mettre en œuvre le procédé tel que décrit en référence à la figure 3.

Ce dispositif 10 comporte une entrée 11 pour recevoir un gaz combustible G1 et une entrée 12 pour recevoir un gaz de référence G2 (typiquement du méthane).

Le dispositif 10 comporte également un module de sélection et de guidage pour amener le flux du gaz combustible ou du gaz de référence à l'entrée d'une conduite 13. Le module de sélection et de guidage comporte en fait une vanne 31 et une vanne 35.

Les vannes 31 et 35 sont commandées respectivement par un module 36 par ses bornes 52 et 51 pour faire circuler soit le gaz combustible soit le gaz de référence dans la conduite.

La conduite comporte ici, d'amont en aval depuis son entrée 13 :

- Un capteur de pression absolue 60 connecté au module 36 par sa borne 41,
- Un capteur de température absolue 50 connecté au module 36 par sa borne 42,
- Une restriction fluidique 32 (par exemple un orifice ou une micro-tuyère),
- Un capteur de débit massique thermique 33 connecté au module 36 par sa borne 43,
- Un capteur de fraction molaire de dihydrogène 34 connecté au module 36 par sa borne 44,

Le module 36 peut appliquer une loi affine empirique telles que celles présentées ci-avant pour estimer l'indice de Wobbe à partir des signaux reçus aux bornes 41 à 44.

Dans l'exemple illustré, le module 36 communique par deux bornes de sorties 54 et 53 à un module externe 37, par exemple un actionneur pour mettre en œuvre une régulation, ou encore un afficheur. Dans une variante non illustrée, le module 37 est intégré au dispositif 10.

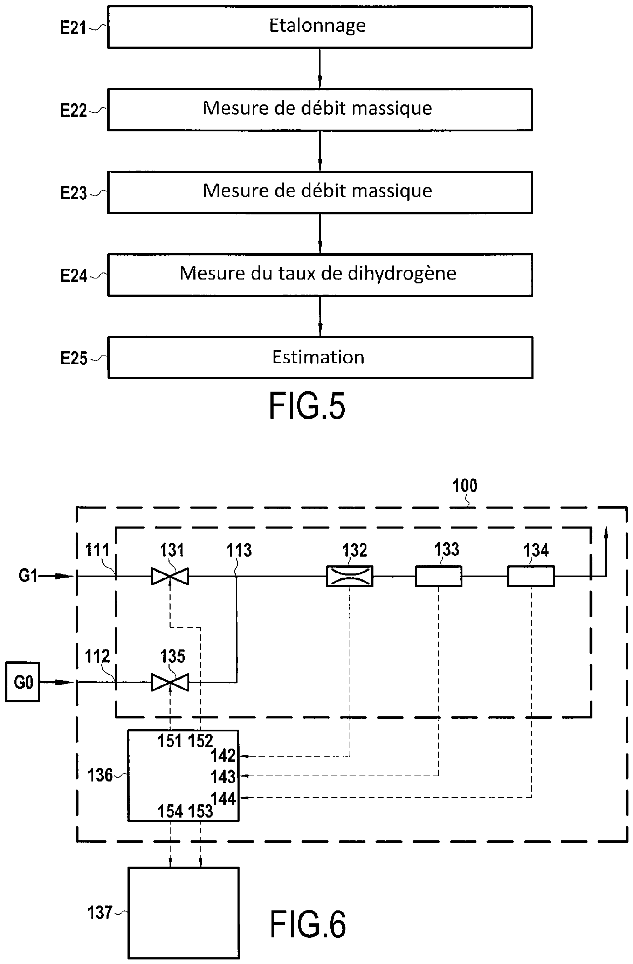

On va maintenant décrire, en se référant aux figures 5 et 6, un mode de mise en œuvre et de réalisation dans lequel on estime le pouvoir calorifique supérieur d'un gaz combustible.

Sur la figure 5, on a représenté les étapes d'un procédé d'estimation du pouvoir calorifique supérieur $PCS_{GN/H2}$ d'un gaz combustible.

Dans une étape E22, on mesure le débit massique dudit gaz combustible en écoulement laminaire à travers un appareil faisant apparaître une chute de pression, cette mesure dépendant de la viscosité du gaz combustible et de la viscosité d'un gaz de référence.

Dans une étape E23, en aval dudit appareil faisant apparaître une chute de pression, on mesure le débit massique dudit gaz combustible au moyen d'un débitmètre massique thermique, la mesure dépendant de la capacité thermique massique du gaz combustible et de la capacité thermique du gaz de référence.

Dans une étape E21, qui peut être mise en œuvre antérieurement ou postérieurement à la mise en œuvre des étapes E22 et E23, on met en œuvre un étalonnage qui correspond à la mise en œuvre des étapes E22 et E23 mais avec un gaz de référence (par exemple du méthane).

On met également en œuvre une étape E24 de mesure du taux de dihydrogène $X_{H2}$, en fraction molaire.

Enfin, dans une étape E25, on met en œuvre une estimation du pouvoir calorifique supérieur au moyen d'une loi affine empirique de la forme :

$$PCS_{GN/H2} = A + B \cdot Z + C \cdot X_{H_2}$$

avec :

$$Z = \frac{Q_{mes,1}}{Q_{mes,2}}$$

où Z est une variable correspondant à la variable Y décrite en référence à la figure 1,

$Q_{mes,1}$ est le débit massique du gaz combustible en écoulement laminaire à travers un appareil faisant apparaître une chute de pression mesuré, et

$Q_{mes,2}$ est le débit massique du gaz combustible mesuré en aval dudit appareil faisant apparaître une chute de pression ; et

A, B, et C sont des coefficients prédéterminés pour la famille de gaz combustibles et correspondant respectivement aux coefficients $\alpha$, $\beta$, et $\gamma$.

Du document antérieur DE 4 118 781, on connait la relation suivante sur le pouvoir calorifique supérieur (*PCS*):

$$PCS = \alpha \cdot \left( \frac{\rho C_p}{\mu} \right) + \beta$$

Avec :

$\alpha$ et $\beta$ des constantes prédéterminées,

$\rho$ la masse volumique du gaz combustible,

$\mu$ la viscosité du gaz combustible, et

$C_p$ la capacité calorifique du gaz combustible.

On peut définir la variable sans dimensions utilisée ci-dessus Z de la manière suivante :

$$Z = \frac{(\rho C_p / \mu)_{GN}}{(\rho C_p / \mu)_{ref}}$$

Ici, l'indice GN vise le gaz combustible, et ref vise un gaz de référence.

On peut réécrire la relation sur le pouvoir calorifique supérieur de la manière suivante :

$$PCS_{GN} = A \cdot Z + B$$

Pour estimer Z, on utilise les deux mesures de débit. Par exemple, $Q_{mes,1}$ est le débit massique du gaz combustible en écoulement laminaire à travers un appareil faisant apparaître une chute de pression mesuré. En utilisant la loi de Poiseuille, on sait que l'appareil fait subir au flux gazeux une chute de pression à travers un élément laminaire.

La mesure dépendant de la viscosité, pour certains appareils de mesure, elle aura une forme dépendant de la différence de pression amont-aval $\Delta p_{mes}$ et le débitmètre qui délivre $Q_{mes,1}$ peut déterminer le débit volumique au moyen de la formule suivante :

$$Q_{vol,1} = K_1 \cdot \frac{\Delta p_{mes}}{\mu}$$

Avec $K_1$ une constante géométrique.

Ici, un capteur de pression et une sonde de température sont intégrés au débitmètre et ils permettent de donner directement le débit massique (ou volumique corrigé aux conditions normales de température et pression) $Q_{mes,1}$ :

$$Q_{mes,1}[Nm^3/h] = Q_{vol,1}[m^3/h] \cdot \frac{p_{mes}}{p_0} \cdot \frac{T_0}{T_{mes}}$$

La mesure de débit volumique corrigé $Q_{mes,1}$ dépend de la viscosité du combustible gazeux.

La mesure du débit $Q_{mes,2}$ est obtenue à l'aide d'un débitmètre massique thermique. La mesure de débit massique $Q_{mes,2}$ dépend de la capacité calorifique du combustible gazeux.

Ainsi, on obtient deux estimations du débit massique, chacune effectuant présentant une erreur par rapport au débit volumique normal réel ($Q_{vn}$). On a :

$$Q_{mes,1} = Q_{vn} \frac{\mu_{GN}}{\mu_{ref}}$$

$$Q_{mes,2} = Q_{vn} \frac{(\rho C_p)_{GN}}{(\rho C_p)_{ref}}$$

Pour $Q_{mes,1}$, l'erreur est liée à l'écart entre la viscosité réelle du gaz de mélange et la viscosité du gaz de référence (ici du méthane).

Pour $Q_{mes,2}$, l'erreur est liée à l'écart entre la masse volumique multipliée par la capacité calorifique massique réelle du gaz de mélange, et la même quantité pour le gaz de référence (ici du méthane).

En faisant le rapport des deux débits mesurés, on détermine la variable Z et la loi de corrélation du document DE 4 118 781 devient :

$$PCS_{GN} = A \cdot \frac{Q_{mes,1}}{Q_{mes,2}} + B$$

En notant :

$U_{mes,1} = Q_{mes,1}$, et

$U_{mes,2} = Q_{mes,2}$

On a :

$$Z = f\left(U_{mes,1}; U_{mes,2}\right) = \frac{Q_{mes,1}}{Q_{mes,2}}$$

Cela étant, les inventeurs ont observé que cette relation n'est pas applicable dès lors que du dihydrogène est présent dans le gaz combustible. En effet, l'erreur sur la mesure devient trop importante car le dihydrogène a des propriétés (viscosité, capacité thermique) très différentes de celles des gaz tels que les alcanes.

Pour pallier cet inconvénient, on utilise la mesure du taux de dihydrogène $X_{H2}$, et il est proposé l'équation suivante :

$$PCS_{GN/H2} = A + B \cdot Z + C \cdot X_{H_2}$$

$A$, $B$, et $C$ étant des coefficients prédéterminés pour la famille de gaz combustibles et correspondant respectivement aux coefficients $\alpha$, $\beta$, et $\gamma$ décrits en référence aux figures 1 et 2.

Les coefficients $A$, $B$, et $C$ peuvent être obtenus à partir de composition de gaz connues, par exemple des compositions de gaz naturels du réseau d'un pays ou d'une région. Par exemple, on peut utiliser des compositions connues de gaz à haut pouvoir calorifique distribué en Europe et bien connu de l'homme du métier.

A partir de ces compositions connues, on peut définir les valeurs limites sur la fraction molaire des différents composés. Par exemple, en notant XK la fraction molaire de l'espèce K dans un gaz, on peut avoir des compositions connues de ce type :

0% < XN2 < 5.5%

0.5 < XC2H6 < 12.5%

0% < XC4H10 < 3.5%

0% < XCO2 < 1.3%

0% < XC3H8 < 3.5%

0% < XC5H12 < 3.5%

$$XCH4 = 1 - \cdot X\_k$$

On notera qu'il n'y a pas de dihydrogène dans les compositions actuelles de gaz.

Ces gammes possibles permettent de générer aléatoirement des compositions de gaz pour lesquelles on peut déterminer le pouvoir calorifique supérieur. 10000 gaz peuvent ainsi être générés.

On peut, pour chaque gaz généré aléatoirement, ajouter une quantité aléatoire de dihydrogène comprise entre 0% et 20% de fraction molaire. Ici encore, on peut déterminer le pouvoir calorifique supérieur de ces gaz générés aléatoirement.

On peut également déduire des compositions générées aléatoirement les valeurs associées de la variable $Z$. En appliquant une méthode des moindres carrés on peut obtenir des valeurs pour les coefficients $A$, $B$, et $C$ :

A = 0,61650996

B = 10.428

C = -0,0645996

De manière alternative, on peut utiliser une corrélation dans laquelle les coefficients ne sont pas des constantes et dépendent du taux de dihydrogène en fraction molaire.

On peut réécrire la loi affine empirique de la manière suivante :

$$PCS_{GN/H2} = A\left(X_{H_2}\right) + B\left(X_{H_2}\right) \cdot Z$$

Ici, $A$ et $B$ sont des fonctions du taux de dihydrogène (l'équation a été réécrite pour ne faire apparaître que deux coefficients qui dépendent de $X_{H_2}$, toutefois, il est possible d'écrire l'équation avec trois coefficients).

De la même manière, en générant des compositions aléatoires de gaz, les inventeurs ont observé qu'il est possible d'utiliser des fonctions A et *B* constantes par morceau sur des intervalles de concentration en dihydrogène de largeur 1%.

Par exemple, entre 19% et 20% de dihydrogène, *A* et *B* prennent les valeurs suivantes :

$A$ = 0.397217

$B$ = 10.189715

Sur la figure 6, on a représenté un dispositif d'estimation du pouvoir calorifique supérieur, pouvant mettre en œuvre le procédé tel que décrit en référence à la figure 5.

Ce dispositif 100 comporte une entrée 111 pour recevoir un gaz combustible G1 et une entrée 112 pour recevoir un gaz de référence G2 (typiquement du méthane).

Le dispositif 100 comporte également un module de sélection et de guidage pour amener le flux du gaz combustible ou du gaz de référence à l'entrée d'une conduite 113. Le module de sélection et de guidage comporte en fait une vanne 131 et une vanne 135.

Les vannes 131 et 135 sont commandées respectivement par un module 136 par ses bornes 152 et 151 pour faire circuler soit le gaz combustible soit le gaz de référence dans la conduite.

La conduite comporte ici, d'amont en aval depuis son entrée 113 :

- Un capteur 132 de débit massique en écoulement laminaire à travers un appareil faisant apparaître une chute de pression, la mesure dépendant de la viscosité du gaz combustible et de la viscosité d'un gaz de référence, ce capteur étant connecté au module 136 par sa borne 142,
- Un capteur 133 de débit massique thermique, la mesure dépendant de la capacité thermique massique du gaz mesuré et de la capacité thermique du gaz de référence, ce capteur étant connecté au module 136 par sa borne 143,
- Un capteur de fraction molaire de dihydrogène 134 connecté au module 136 par sa borne 144,

Le module 136 peut appliquer une loi affine empirique telles que celles présentées ci-avant pour estimer le pouvoir calorifique supérieur à partir des signaux reçus aux bornes 142 à 144.

Dans l'exemple illustré, le module 136 communique par deux bornes de sorties 154 et 153 à un module externe 137, par exemple un actionneur pour mettre en œuvre une régulation, ou encore un afficheur. Dans une variante non illustrée, le module 137 est intégré au dispositif 100.

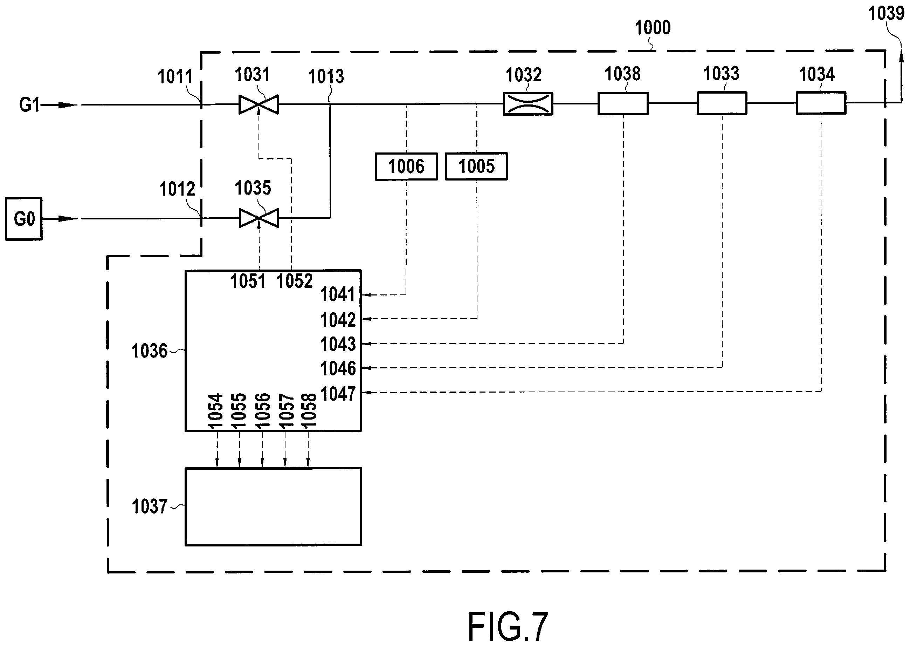

La figure 7 est un exemple d'un dispositif pouvant à la fois déterminer le pouvoir calorifique supérieur et l'indice de Wobbe au moyen de deux lois affines empiriques.

Ce dispositif comporte deux arrivées de gaz sous pression. Une première entrée 1011 est destinée à recevoir un gaz combustible G1 appartenant à une famille de gaz (par exemple du gaz combustible de la deuxième famille selon la norme française NF EN 437) et contenant en plus du dihydrogène. Le dispositif 1000 comporte une autre entrée1012 pour un gaz de référence G0 utilisé pour les phases d'étalonnage.

Le dispositif 1000 comporte également un module de sélection et de guidage pour amener le flux du gaz combustible G1 ou du gaz de référence G0 à l'entrée d'une conduite 1013. Le module de sélection et de guidage comporte en fait une vanne 1031 et une vanne 1035.

Les vannes 1031 et 1035 sont commandées respectivement par un module 1036 par ses bornes 1052 et 1051 pour faire circuler soit le gaz combustible soit le gaz de référence dans la conduite.

A partir de l'entrée de la conduite 1013, d'amont en aval, on a :

- Un capteur de pression absolue 1006 connecté au module 1036 par sa borne 1041. Ce capteur délivre la valeur $p_{mes}$ mesurée sur le gaz combustible et la valeur $p_{ref}$ mesurée sur le gaz de référence.
- Un capteur de température absolue 1005 connecté au module 1036 par sa borne 1042. Ce capteur délivre la valeur $T_{mes}$ mesurée sur le gaz combustible et la valeur $T_{ref}$ mesurée sur le gaz de référence.
- Une restriction fluidique 1032 (par exemple un orifice ou une micro-tuyère),
- Un capteur de débit massique à travers une chute de pression laminaire connecté au module 1036 par sa borne 1043. Ce capteur délivre la valeur $Q_{mes,1}$.
- Un capteur de débit massique thermique 1033 connecté au module 1036 par sa borne 1046. Ce capteur délivre la valeur $Q_{mes,2}$ mesurée sur le gaz combustible et la valeur $Q_{ref}$ mesurée sur le gaz de référence.
- Un capteur de fraction molaire de dihydrogène 1034 connecté au module 36 par sa borne 1047,
- Un évent (39).

Ici, le module 1036 peut calculer les deux variables suivantes :

$$Y = \left(\frac{p_{ref}}{p_{mes}} \cdot \sqrt{\frac{T_{ref}}{T_{mes}}}\right) \cdot \left(\frac{Q_{mes,2}}{Q_{ref}}\right)$$

$$Z = \frac{Q_{mes,2}}{Q_{mes,1}}$$

Le module 1036 peut ensuite utiliser les formules suivantes pour déterminer l'indice de Wobbe $IW_{GN/H2}$, le pouvoir calorifique supérieur $PCS_{GN/H2}$, le volume d'air stœchiométrique $V_{a_{GN/H2}}$, l'indice de comburité $B_{GN/H2}$ et la densité du mélange de gaz combustible $d_{GN/H2}$ :

$$IW_{GN/H2} = D + E \cdot Y + F \cdot X_{H_2}$$

$$PCS_{GN/H2} = A + B \cdot Z + C \cdot X_{H_2}$$

$$d_{GN/H2} = \left(\frac{PCS_{GN/H2}}{IW_{GN/H2}}\right)^2$$

$$V_{a_{GN/H2}} = \frac{PCS_{GN/H2}}{0.000953 \cdot X_{H_2} + 1,165475}$$

$$B_{GN/H2} = \frac{IW_{GN/H2}}{0.000953 \cdot X_{H_2} + 1,165475}$$

En effet, il est possible de déterminer le volume d'air stœchiométrique Va, l'indice de comburité B et la densité du mélange de gaz combustible à partir des valeurs de l'indice de Wobbe IW et du pouvoir calorifique supérieur PCS.

Plus précisément, pour les gaz naturels habituellement distribués en Europe (qui ne comportent pas actuellement du dihydrogène), on a :

$$\frac{PCS}{Va} = \frac{IW}{B} = 1,162$$

Les inventeurs ont observé qu'en présence de dihydrogène, on peut s'écarter de la valeur 1,162.

On peut lier le rapport entre le pouvoir calorifique supérieur et le volume d'air stœchiométrique au taux de dihydrogène (connu ici), et ce avec une erreur de moins de 0,3%, pour tout gaz naturel étudié.

En d'autres termes, la connaissance du taux de dihydrogène et l'estimation avec une erreur de moins de 1% du pouvoir calorifique supérieur permettent de connaitre le volume d'air stœchiométrique et donc d'effectuer des réglages de combustion en boucle de régulation ouverte.

De la même manière, le rapport entre l'indice de Wobbe et l'indice de comburité évolue linéairement avec le taux de dihydrogène. Ceci permet également de mettre en œuvre une régulation en boucle ouverte.

On peut noter que l'erreur dans l'estimation de l'indice de comburité est inférieure à 1% pour 99,5% des 10000 gaz de composition aléatoires si 10000 gaz sont utilisés.

De par la définition de l'indice de Wobbe, on peut déterminer la densité du gaz dès lors que l'on connaît l'indice de Wobbe et le pouvoir calorifique supérieur.

On peut noter que le dispositif 1000 délivre en sa borne 1054 un signal d'indice de Wobbe, en sa borne 1055 un signal de pouvoir calorifique supérieur, en sa borne 1056 un signal de densité, en sa borne 1057 un signal de volume d'air stœchiométrique, et en sa borne 1058 un signal d'indice de comburité. Ces signaux concernent le gaz combustible d'étude, c'est-à-dire le gaz G1.

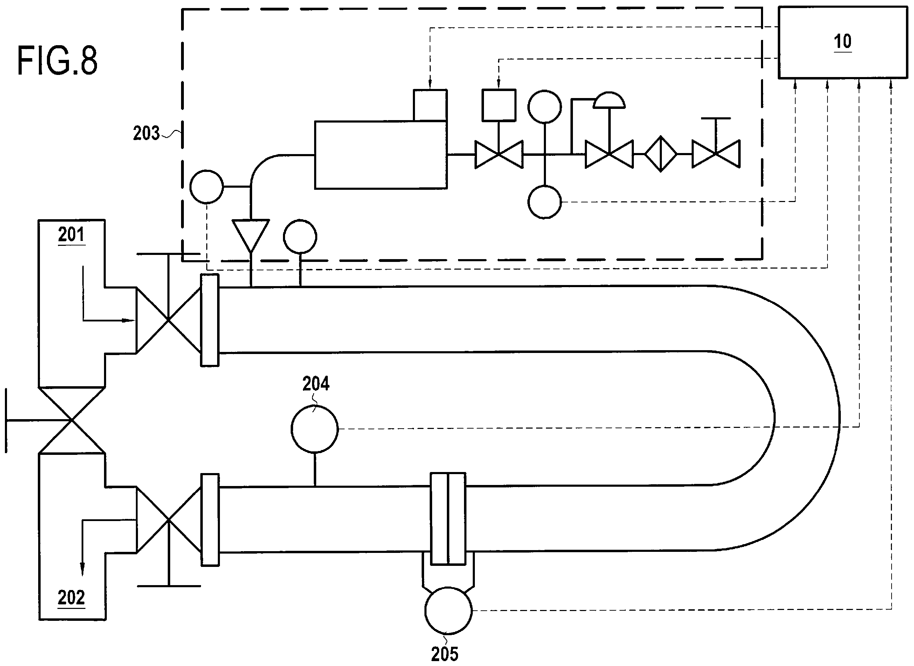

Sur la figure 8, on a représenté un exemple d'application de régulation utilisant le dispositif 10 décrit en référence à la figure 4. Ce dispositif est raccordé entre deux points d'une conduite, un point d'entrée 201 et un point de sortie 202. Le dispositif 10 commande un module de fourniture d'air comprimé 203 en fonction des mesures effectuées par les capteurs 205.

Cet appareil peut notamment réguler de manière continue l'indice de Wobbe pour des gaz analogues à ceux distribués en Europe avec une quantité supplémentaire de dihydrogène. C'est notamment parce que la mesure est continue ou en temps réel que la régulation est possible alors que tel n'est pas le cas avec les appareils de type chromatographe.

La précision de la mesure de l'indice de Wobbe est de l'ordre de 1%.

On peut noter qu'il est possible de modifier une valeur de consigne de l'indice de Wobbe dans le dispositif 10 pour obtenir la régulation désirée.

En outre, les étapes d'étalonnages peuvent être réalisées de manière automatique en utilisant du méthane. Les phases de démarrage peuvent également être mises en œuvre de manière automatique, c'est-à-dire sans intervention d'un opérateur.

Aussi, une régulation mixte est possible dans laquelle on met en œuvre une régulation en boucle fermée sur l'une des caractéristique (par exemple l'indice de Wobbe) et en boucle ouverte sur un débit d'air comprimé à injecter. Cela peut permettre de prendre en compte de manière particulièrement précise les variations de la caractéristique pour mieux respecter une consigne de caractéristique.

On peut noter qu'un tel appareil peut avoir une consommation de gaz naturel inférieure à 150 litres par heure.

Les modes de mise en œuvre et de réalisation décrits ci-avant permettent de coupler des données thermodynamiques (Cp, viscosité) avec une mesure du taux de dihydrogène en volume ou en fraction molaire. L'utilisation d'une corrélation appropriée permet de calculer l'indice de Wobbe et/ou le pouvoir calorifique supérieur.

On peut noter que par rapport à l'utilisation d'un chromatographe en phase gazeuse, on a :

- une estimation presque instantanée (avec un temps de réponse qui peut être inférieur à 5 secondes),
- une estimation peu coûteuse,

Par rapport aux méthodes à corrélation existantes, on a une bonne précision alors que du dihydrogène est présent, et une bonne robustesse de mesure.

Enfin, par rapport à des appareils effectuant une combustion (comburimètre ou calorimètre), on a une bonne robustesse, une facilité de mise en œuvre, et une maintenance réduite et facile à réaliser.

**Revendications**

1. Procédé d'estimation d'au moins une caractéristique de combustion d'un gaz combustible appartenant à une famille de gaz combustibles, ladite au moins une caractéristique étant l'indice de Wobbe ou le pouvoir calorifique supérieur, le procédé comprenant :

   une mesure (E01) d'au moins deux propriétés d'écoulement dudit gaz combustible, les propriétés d'écoulement du gaz combustible étant des propriétés mesurées dans un organe d'écoulement et qui sont choisies dans le groupe comprenant le débit, la température, et la pression,
   une mesure (E02) du taux $X_{H2}$ de dihydrogène contenu dans ledit gaz combustible, le procédé étant **caractérisé en ce que** ladite au moins une caractéristique $\Xi_{GN/H2}$ est estimée (E03) au moyen
   de la loi affine empirique suivante :

   $$\Xi_{GN/H2} = \alpha + \beta \cdot Y + \gamma \cdot X_{H_2}$$

   avec $\alpha$, $\beta$, et $\gamma$ des coefficients prédéterminés pour la famille de gaz combustibles, et
   Y une variable représentative de propriétés physiques dudit gaz combustible élaborée à partir desdites valeurs mesurées desdites au moins deux propriétés d'écoulement dudit gaz combustible, Y étant une variable représentative de propriétés physiques du gaz combustible telles que la viscosité, la capacité calorifique massique, ou encore la densité, et exprimable en fonction des valeurs mesurées des propriétés d'écoulement du gaz combustible.

**2.** Procédé selon la revendication 1, dans lequel les coefficients $\alpha$, $\beta$, et $\gamma$ sont des coefficients lus dans une cartographie ayant pour entrée la valeur mesurée du taux $X_{H2}$ de dihydrogène et délivrant en sortie lesdits coefficients $\alpha$, $\beta$, et $\gamma$.

**3.** Procédé selon la revendication 2, dans lequel ladite cartographie associe à des gammes de valeurs larges de 1% de taux $X_{H_2}$ de dihydrogène lesdits coefficients $\alpha$, $\beta$, et $\gamma$.

**4.** Procédé selon l'une quelconque des revendications 1 à 3, dans lequel les valeurs desdits coefficients $\alpha$, $\beta$, et $\gamma$ sont obtenues à partir d'un ensemble de données relatives à des gaz connus de ladite famille de gaz combustibles pour lesquels on connaît les caractéristiques de combustion et la valeur de Y représentatives desdites propriétés physiques.

**5.** Procédé selon la revendication 4, comprenant une génération aléatoire de caractéristiques de combustion et de valeurs de Y représentatives de propriétés physiques à partir dudit ensemble de données relatives à des gaz connus de ladite famille de gaz combustibles.

**6.** Procédé selon l'une quelconque des revendications 1 à 5, on estime l'indice de Wobbe et le pouvoir calorifique supérieur au moyen de deux lois affines empiriques.

**7.** Procédé selon la revendication 6, comprenant en outre une estimation de la densité dudit gaz combustible à partir de l'indice de Wobbe estimé et du pouvoir calorifique supérieur estimé.

**8.** Procédé selon l'une quelconque des revendications 1 à 7, comprenant une régulation de la caractéristique de combustion du gaz combustible ou une régulation de la caractéristique de combustion d'un gaz combustible et d'un volume d'air stœchiométrique estimé ou d'un indice de comburité estimé correspondant à ladite caractéristique estimée.

**9.** Procédé selon l'une quelconque des revendications 1 à 8, dans lequel ladite au moins une caractéristique de combustion du gaz combustible comprend l'indice de Wobbe $IW_{GN/H2}$,

ladite mesure d'au moins deux propriétés d'écoulement dudit gaz combustible comporte une mesure (E12) d'un débit massique du gaz combustible en écoulement sonique à travers une restriction fluidique, effectuée à une pression absolue mesurée en amont de la restriction et à une température absolue mesurée en amont de la restriction,
le procédé comprenant en outre une procédure d'étalonnage (E11) au cours de laquelle est effectuée une mesure d'un débit massique d'un gaz de référence en écoulement sonique à travers ladite restriction fluidique, à une pression absolue de référence mesurée et à une température absolue de référence mesuré ;
la loi affine empirique utilisée pour estimer (E14) l'indice de Wobbe $IW_{GN/H2}$ s'écrivant alors :

$$IW_{GN/H2} = D + E \cdot Y + F \cdot X_{H_2}$$

avec :

$$Y = \frac{Q_{mes,2}}{Q_{ref}} \cdot \frac{p_{ref}}{p_{mes}} \cdot \sqrt{\frac{T_{ref}}{T_{mes}}}$$

où $Q_{mes,2}$ est le débit massique du gaz combustible mesuré,

$p_{mes}$ est la pression absolue du gaz combustible mesurée,
$T_{mes}$ est la température absolue du gaz combustible mesurée,
$Q_{ref}$ est le débit massique du gaz de référence mesuré,
$p_{ref}$ est la pression absolue du gaz de référence mesurée, et
$T_{ref}$ est la température absolue du gaz de référence mesurée ; et *D, E,* et *F* sont des coefficients prédéterminés pour la famille de gaz combustibles et correspondant respectivement aux coefficients $\alpha$, $\beta$, et $\gamma$.

**10.** Procédé selon l'une quelconque des revendications 1 à 9, dans lequel on a estimé l'indice de Wobbe dudit gaz combustible, le procédé comprenant en outre une mesure de la densité dudit gaz combustible et une estimation du pouvoir calorifique supérieur à partir de l'indice de Wobbe estimé et de la densité du gaz mesurée.

11. Procédé selon l'une quelconque des revendications 1 à 9, dans lequel ladite au moins une caractéristique de combustion du gaz combustible comprend le pouvoir calorifique supérieur $PCS_{GN/H2}$, ladite mesure d'au moins deux propriétés d'écoulement dudit gaz combustible comporte :

une mesure (E22) de débit massique dudit gaz combustible en écoulement laminaire à travers un appareil faisant apparaître une chute de pression, la mesure dépendant de la viscosité du gaz combustible et de la viscosité d'un gaz de référence, et
une mesure (E23), en aval dudit appareil faisant apparaître une chute de pression, du débit massique dudit gaz combustible au moyen d'un débitmètre massique thermique, la mesure dépendant de la capacité thermique massique du gaz combustible et de la viscosité d'un gaz de référence ;
la loi affine empirique utilisée pour estimer (E25) le pouvoir calorifique supérieur $PCS_{GN/H2}$ s'écrivant alors

$$PCS_{GN/H2} = A + B \cdot Z + C \cdot X_{H_2}$$

avec :

$$Z = \frac{Q_{mes,1}}{Q_{mes,2}}$$

où $Z$ est une variable correspondant à la variable $Y$,

$Q_{mes,1}$ est le débit massique du gaz combustible en écoulement laminaire à travers un appareil faisant apparaître une chute de pression mesuré, et
$Q_{mes,2}$ est le débit massique du gaz combustible mesuré en aval dudit appareil faisant apparaître une chute de pression ; et
$A$, $B$, et $C$ sont des coefficients prédéterminés pour la famille de gaz combustibles et correspondant respectivement aux coefficients $\alpha$, $\beta$, et $\gamma$.

12. Dispositif d'estimation d'au moins une caractéristique de combustion d'un gaz combustible appartenant à une famille de gaz combustibles, ladite au moins une caractéristique étant l'indice de Wobbe ou le pouvoir calorifique supérieur, le dispositif comprenant :

au moins deux modules de mesure (3a, 3b) d'au moins deux propriétés d'écoulement dudit gaz combustible, les propriétés d'écoulement du gaz combustible étant des propriétés mesurées dans un organe d'écoulement et qui sont choisies dans le groupe comprenant le débit, la température, et la pression,
un module de mesure (4) du taux $X_{H_2}$ de dihydrogène contenu dans ledit gaz combustible, le dispositif étant **caractérisé par** un module (5) configuré pour estimer ladite au moins une caractéristique $\Xi_{GN/H2}$ au moyen de la loi affine empirique suivante :

$$\Xi_{GN/H2} = \alpha + \beta \cdot Y + \gamma \cdot X_{H_2}$$

avec $\alpha$, $\beta$, et $\gamma$ des coefficients prédéterminés pour la famille de gaz combustibles, et
$Y$ une variable représentative de propriétés physiques dudit gaz combustible élaborée à partir desdites valeurs mesurées desdites au moins deux propriétés d'écoulement dudit gaz combustible, $Y$ étant une variable représentative de propriétés physiques du gaz combustible telles que la viscosité, la capacité calorifique massique, ou encore la densité, et exprimable en fonction des valeurs mesurées des propriétés d'écoulement du gaz combustible.

13. Dispositif selon la revendication 12, dans lequel ladite au moins une caractéristique de combustion du gaz combustible comprend l'indice de Wobbe $IW_{GN/H2}$, le dispositif comprenant :

une entrée (11) pour recevoir un flux dudit gaz combustible,

une entrée (12) pour recevoir un flux d'un gaz de référence,
un module de sélection et de guidage (31, 35) pour amener le flux dudit gaz combustible ou le flux dudit gaz de référence à une conduite (13),
une restriction fluidique (32),
un module de mesure (33) d'un débit massique du gaz combustible en écoulement sonique à travers ladite restriction fluidique, comprenant un sous-module de mesure de la pression absolue en amont de la restriction et un sous-module de mesure de la température absolue en amont de la restriction,
la loi affine empirique utilisée pour estimer l'indice de Wobbe $IW_{GN/H2}$ s'écrivant alors :

$$IW_{GN/H2} = D + E \cdot Y + F \cdot X_{H_2}$$

avec :

$$Y = \frac{Q_{mes,2}}{Q_{ref}} \cdot \frac{p_{ref}}{p_{mes}} \cdot \sqrt{\frac{T_{ref}}{T_{mes}}}$$

où $Q_{mes,2}$ est le débit massique du gaz combustible mesuré,

$p_{mes}$ est la pression absolue du gaz combustible mesurée,
$T_{mes}$ est la température absolue du gaz combustible mesurée,
$Q_{ref}$ est le débit massique du gaz de référence mesuré,
$p_{ref}$ est la pression absolue du gaz de référence mesurée, et
$T_{ref}$ est la température absolue du gaz de référence mesurée ; et D, E, et F sont des coefficients prédéterminés pour la famille de gaz combustibles et correspondant respectivement aux coefficients $\alpha$, $\beta$, et $\gamma$.

**14.** Dispositif selon la revendication 12 ou 13, dans lequel le dispositif est apte à estimer l'indice de Wobbe dudit gaz combustible, le dispositif comprenant en outre un module de mesure de la densité dudit gaz combustible et le module (5) configuré pour estimer ladite au moins une caractéristique étant en outre configuré pour estimer le pouvoir calorifique supérieur à partir de l'indice de Wobbe estimé et de la densité du gaz mesurée.

**15.** Dispositif selon la revendication 12 ou 13, dans lequel ladite au moins une caractéristique de combustion du gaz combustible comprend le pouvoir calorifique supérieur $PCS_{GN/H2}$, le dispositif comprenant :

une entrée (111) pour recevoir un flux dudit gaz combustible,
un module de mesure (132) de débit massique dudit gaz combustible en écoulement laminaire à travers un appareil faisant apparaître une chute de pression, la mesure dépendant de la viscosité du gaz combustible et de la viscosité d'un gaz de référence, et
un module de mesure (133), en aval dudit appareil faisant apparaître une chute de pression, du débit massique dudit gaz combustible au moyen d'un débitmètre massique thermique, la mesure dépendant de la capacité thermique massique du gaz combustible et de la capacité thermique d'un gaz de référence ;
la loi affine empirique utilisée pour estimer le pouvoir calorifique supérieur $PCS_{GN/H2}$ s'écrivant alors

$$PCS_{GN/H2} = A + B \cdot Z + C \cdot X_{H_2}$$

avec :

$$Z = \frac{Q_{mes,1}}{Q_{mes,2}}$$

où Z est une variable correspondant à la variable Y,
$Q_{mes,1}$ est le débit massique du gaz combustible en écoulement laminaire à travers un appareil faisant apparaître une chute de pression mesuré, et
$Q_{mes,2}$ est le débit massique du gaz combustible mesuré en aval dudit appareil faisant apparaître une chute de pression ; et

A, B, et C sont des coefficients prédéterminés pour la famille de gaz combustibles et correspondant respectivement aux coefficients α, β, et γ.

16. Dispositif selon l'une des revendications 12 à 15, dans lequel le module configuré pour estimer ladite au moins une caractéristique $\Xi_{GN/H2}$ au moyen de la loi affine empirique est en outre configuré pour estimer un volume d'air stœchiométrique ou un indice de comburité.

17. Dispositif selon l'une des revendications 12 à 16, comprenant en outre un module de régulation de ladite caractéristique de combustion du gaz combustible ou de régulation de la caractéristique de combustion d'un gaz combustible et d'un volume d'air stœchiométrique estimé ou d'un indice de comburité estimé correspondant à ladite caractéristique estimée.

**Patentansprüche**

1. Verfahren zur Schätzung mindestens eines Verbrennungsmerkmals eines brennbaren Gases, das zu einer Familie von brennbaren Gasen gehört, wobei das mindestens eine Merkmal der Wobbeindex oder der obere Heizwert ist, wobei das Verfahren umfasst:

   eine Messung (E01) von mindestens zwei Strömungseigenschaften des brennbaren Gases, wobei die Strömungseigenschaften des brennbaren Gases Eigenschaften sind, die in einem Strömungsorgan gemessen werden und die aus der Gruppe umfassend den Durchfluss, die Temperatur und den Druck ausgewählt sind, eine Messung (E02) des Gehalts $X_{H2}$ an Dihydrogen, das in dem brennbaren Gas enthalten ist, wobei das Verfahren **dadurch gekennzeichnet ist, dass**
   das mindestens eine Merkmal $\Xi_{GN/H2}$ mittels des folgenden empirischen affinen Gesetzes geschätzt (E03) wird:

   $$\Xi_{GN/H2} = \alpha + \beta \cdot Y + \gamma \cdot X_{H_2},$$

   wobei α, β und γ vorher bestimmte Koeffizienten für die Familie von brennbaren Gasen sind, und
   Y eine Variable ist, die physikalische Eigenschaften des brennbaren Gases darstellt, und die aus den Messwerten der mindestens zwei Strömungseigenschaften des brennbaren Gases erstellt wird, wobei Y eine Variable ist, die physikalische Eigenschaften des brennbaren Gases wie die Viskosität, die spezifische Wärme oder auch die Dichte darstellt und in Abhängigkeit von Messwerten von Strömungseigenschaften des brennbaren Gases ausdrückbar ist.

2. Verfahren nach Anspruch 1, wobei die Koeffizienten α, β und γ Koeffizienten sind, die aus einer Kartierung gelesen werden, die den Messwert des Gehalts $X_{H2}$ an Dihydrogen als Eingang aufweist und die Koeffizienten α, β und γ als Ausgang liefert.

3. Verfahren nach Anspruch 2, wobei die Kartierung die Koeffizienten α, β und γ mit breiten Wertebereichen von 1 % des Gehalts $X_{H2}$ an Dihydrogen assoziiert.

4. Verfahren nach einem der Ansprüche 1 bis 3, wobei die Werte der Koeffizienten α, β und γ aus einem Datensatz in Bezug auf bekannte Gase der Familie von brennbaren Gasen, für die die Verbrennungsmerkmale und der Wert von Y, die die physikalischen Eigenschaften darstellen, bekannt sind, erhalten werden.

5. Verfahren nach Anspruch 4, umfassend eine Zufallserzeugung von Verbrennungsmerkmalen und Werten von Y, die physikalische Eigenschaften darstellen, aus dem Datensatz in Bezug auf bekannte Gase der Familie von brennbaren Gasen.

6. Verfahren nach einem der Ansprüche 1 bis 5, wobei der Wobbeindex und der obere Heizwert mittels zweier empirischer affiner Gesetze geschätzt werden.

7. Verfahren nach Anspruch 6, ferner umfassend eine Schätzung der Dichte des brennbaren Gases aus dem geschätzten Wobbeindex und dem geschätzten oberen Heizwert.

8. Verfahren nach einem der Ansprüche 1 bis 7, umfassend eine Regelung des Verbrennungsmerkmals des brennbaren Gases oder eine Regelung des Verbrennungsmerkmals eines brennbaren Gases und eines geschätzten stöchiometrischen Luftvolumens oder eines geschätzten Verbrennungsindexes, entsprechend dem geschätzten Merkmal.

9. Verfahren nach einem der Ansprüche 1 bis 8, wobei das mindestens eine Verbrennungsmerkmal des brennbaren Gases den Wobbeindex $IW_{GN/H2}$ umfasst,

wobei die Messung von mindestens zwei Strömungseigenschaften des brennbaren Gases eine Messung (E12) eines Massendurchflusses des brennbaren Gases in Schallströmung durch eine Fluiddrossel beinhaltet, die bei einem absoluten Druck, der stromaufwärts von der Drossel gemessen wird, und einer absoluten Temperatur, die stromaufwärts von der Drossel gemessen wird, durchgeführt wird,
wobei das Verfahren ferner einen Kalibriervorgang (E11) umfasst, während dem eine Messung eines Massendurchflusses eines Referenzgases in Schallströmung durch die Fluiddrossel bei einem gemessenen absoluten Referenzdruck und bei einer gemessenen absoluten Referenztemperatur durchgeführt wird,
wobei das empirische affine Gesetz, das zum Schätzen (E14) des Wobbeindexes $IW_{GN/H2}$ verwendet wird, dann geschrieben wird als:

$$IW_{GN/H2} = D + E \cdot Y + F \cdot X_{H_2},$$

wobei:

$$Y = \frac{Q_{mes,2}}{Q_{ref}} \cdot \frac{p_{ref}}{p_{mes}} \cdot \sqrt{\frac{T_{ref}}{T_{mes}}},$$

worin $Q_{mes,2}$ der gemessene Massendurchfluss des brennbaren Gases ist,

$p_{mes}$ der gemessene absolute Druck des brennbaren Gases ist,
$T_{mes}$ die gemessene absolute Temperatur des brennbaren Gases ist,
$Q_{ref}$ der gemesssene Massendurchfluss des Referenzgases ist,
$p_{ref}$ der gemessene absolute Druck des Referenzgases ist, und
$T_{ref}$ die gemessene absolute Temperatur des Referenzgases ist, und
$D$, $E$ und $F$ vorher bestimmte Koeffizienten für die Familie von brennbaren Gasen sind und den Koeffizienten $\alpha$, $\beta$ bzw. $\gamma$ entsprechen.

10. Verfahren nach einem der Ansprüche 1 bis 9, wobei der Wobbeindex des brennbaren Gases geschätzt wurde und das Verfahren ferner eine Messung der Dichte des brennbaren Gases und eine Schätzung des oberen Heizwerts aus dem geschätzten Wobbeindex und der gemessenen Dichte des Gases umfasst.

11. Verfahren nach einem der Ansprüche 1 bis 9, wobei das mindestens eine Verbrennungsmerkmal des brennbaren Gases den oberen Heizwert $PCS_{GN/H2}$ umfasst,
wobei die Messung von mindestens zwei Strömungseigenschaften des brennbaren Gases beinhaltet:

eine Messung (E22) eines Massendurchflusses des brennbaren Gases in Laminarströmung durch ein Gerät, das einen Druckabfall hervorbringt, wobei die Messung von der Viskosität des brennbaren Gases und der Viskosität eines Referenzgases abhängt, und
eine Messung (E23) des Massendurchflusses des brennbaren Gases stromabwärts von dem Gerät, das einen Druckabfall hervorbringt, mittels eines thermischen Massendurchflussmessers, wobei die Messung von der spezifischen Wärmekapazität des brennbaren Gases und der Viskosität eines Referenzgases abhängt,
wobei das empirische affine Gesetz, das zum Schätzen (E25) des oberen Heizwerts $PCS_{GN/H2}$ verwendet wird, dann geschrieben wird als

$$PCS_{GN/H2} = A + B \cdot Z + C \cdot X_{H_2},$$

wobei:

$$Z = \frac{Q_{mes,1}}{Q_{mes,2}} \, ,$$

worin Z eine Variable ist, die der Variablen Y entspricht,

$Q_{mes,1}$ der gemessene Massendurchfluss des brennbaren Gases in Laminarströmung durch ein Gerät ist, das einen Druckabfall hervorbringt, und

$Q_{mes,2}$ der Massendurchfluss des brennbaren Gases ist, der stromabwärts von dem Gerät, das einen Druckabfall hervorbringt, gemessen wird, und

A, B und C vorher bestimmte Koeffizienten für die Familie von brennbaren Gasen sind und den Koeffizienten $\alpha$, $\beta$ bzw. $\gamma$ entsprechen.

12. Vorrichtung zur Schätzung mindestens eines Verbrennungsmerkmals eines brennbaren Gases, das zu einer Familie von brennbaren Gasen gehört, wobei das mindestens eine Merkmal der Wobbeindex oder der obere Heizwert ist, wobei die Vorrichtung umfasst:

mindestens zwei Module (3a, 3b) zur Messung von mindestens zwei Strömungseigenschaften des brennbaren Gases, wobei die Strömungseigenschaften des brennbaren Gases Eigenschaften sind, die in einem Strömungsorgan gemessen werden und die aus der Gruppe umfassend den Durchfluss, die Temperatur und den Druck ausgewählt sind,

ein Modul (4) zur Messung des Gehalts $X_{H2}$ an Dihydrogen, das in dem brennbaren Gas enthalten ist, wobei die Vorrichtung **gekennzeichnet ist durch**

ein Modul (5), das dazu ausgestaltet ist, das mindestens eine Merkmal $\Xi_{GN/H2}$ mittels des folgenden empirischen affinen Gesetzes zu schätzen:

$$\Xi_{GN/H2} = \alpha + \beta \cdot Y + \gamma \cdot X_{H_2} \, ,$$

wobei $\alpha$, $\beta$ und $\gamma$ vorher bestimmte Koeffizienten für die Familie von brennbaren Gasen sind, und

Y eine Variable ist, die physikalische Eigenschaften des brennbaren Gases darstellt, und die aus den Messwerten der mindestens zwei Strömungseigenschaften des brennbaren Gases erstellt wird, wobei Y eine Variable ist, die physikalische Eigenschaften des brennbaren Gases wie die Viskosität, die spezifische Wärme oder auch die Dichte darstellt und in Abhängigkeit von Messwerten von Strömungseigenschaften des brennbaren Gases ausdrückbar ist.

13. Vorrichtung nach Anspruch 12, wobei das mindestens eine Verbrennungsmerkmal des brennbaren Gases den Wobbeindex $IW_{GN/H2}$ umfasst, wobei die Vorrichtung umfasst:

einen Eintritt (11) zum Aufnehmen eines Flusses des brennbaren Gases,
einen Eintritt (12) zum Aufnehmen eines Flusses eines Referenzgases,
ein Auswahl- und Führungsmodul (31, 35), um den Fluss des brennbaren Gases oder den Fluss des Referenzgases zu einer Leitung (13) zu lenken,
eine Fluiddrossel (32),
ein Modul (33) zur Messung eines Massendurchflusses des brennbaren Gases in Schallströmung durch die Fluiddrossel, umfassend ein Untermodul zur Messung des absoluten Drucks stromaufwärts von der Drossel und ein Untermodul zur Messung der absoluten Temperatur stromaufwärts von der Drossel,
wobei das empirische affine Gesetz, das zum Schätzen des Wobbeindexes $IW_{GN/H2}$ verwendet wird, dann geschrieben wird als:

$$IW_{GN/H2} = D + E \cdot Y + F \cdot X_{H_2} \, ,$$

wobei:

$$Y = \frac{Q_{mes,2}}{Q_{ref}} \cdot \frac{p_{ref}}{p_{mes}} \cdot \sqrt{\frac{T_{ref}}{T_{mes}}} \, ,$$

worin $Q_{mes,2}$ der gemessene Massendurchfluss des brennbaren Gases ist,

$p_{mes}$ der gemessene absolute Druck des brennbaren Gases ist,
$T_{mes}$ die gemessene absolute Temperatur des brennbaren Gases ist,
$Q_{ref}$ der gemesssene Massendurchfluss des Referenzgases ist,
$p_{ref}$ der gemessene absolute Druck des Referenzgases ist, und
$T_{ref}$ die gemessene absolute Temperatur des Referenzgases ist, und
D, E und F vorher bestimmte Koeffizienten für die Familie von brennbaren Gasen sind und den Koeffizienten $\alpha$, $\beta$ bzw. $\gamma$ entsprechen.

14. Vorrichtung nach Anspruch 12 oder 13, wobei die Vorrichtung dazu geeignet ist, den Wobbeindex des brennbaren Gases zu schätzen, die Vorrichtung ferner ein Modul zur Messung der Dichte des brennbaren Gases umfasst und das Modul (5), das dazu ausgestaltet ist, das mindestens eine Merkmal zu schätzen, ferner dazu ausgestaltet ist, den oberen Heizwert aus dem geschätzten Wobbeindex und der gemessenen Dichte des Gases zu schätzen.

15. Vorrichtung nach Anspruch 12 oder 13, wobei das mindestens eine Verbrennungsmerkmal des brennbaren Gases den oberen Heizwert $PCS_{GN/H2}$ umfasst, wobei die Vorrichtung umfasst:

einen Eintritt (111) zum Aufnehmen eines Flusses des brennbaren Gases,
ein Modul (132) zur Messung eines Massendurchflusses des brennbaren Gases in Laminarströmung durch ein Gerät, das einen Druckabfall hervorbringt, wobei die Messung von der Viskosität des brennbaren Gases und der Viskosität eines Referenzgases abhängt, und
ein Modul (133) zur Messung des Massendurchflusses des brennbaren Gases stromabwärts von dem Gerät, das einen Druckabfall hervorbringt, mittels eines thermischen Massendurchflussmessers, wobei die Messung von der spezifischen Wärmekapazität des brennbaren Gases und der Wärmekapazität eines Referenzgases abhängt,
wobei das empirische affine Gesetz, das zum Schätzen des oberen Heizwerts $PCS_{GN/H2}$ verwendet wird, dann geschrieben wird als

$$PCS_{GN/H2} = A + B \cdot Z + C \cdot X_{H_2}$$

wobei:

$$Z = \frac{Q_{mes,1}}{Q_{mes,2}} \, ,$$

worin Z eine Variable ist, die der Variablen Y entspricht,
$Q_{mes,1}$ der gemessene Massendurchfluss des brennbaren Gases in Laminarströmung durch ein Gerät ist, das einen Druckabfall hervorbringt, und
$Q_{mes,2}$ der Massendurchfluss des brennbaren Gases ist, der stromabwärts von dem Gerät, das einen Druckabfall hervorbringt, gemessen wird, und
A, B und C vorher bestimmte Koeffizienten für die Familie von brennbaren Gasen sind und den Koeffizienten $\alpha$, $\beta$ bzw. $\gamma$ entsprechen.

16. Vorrichtung nach einem der Ansprüche 12 bis 15, wobei das Modul, das dazu ausgestaltet ist, das mindestens eine Merkmal $\Xi_{GN/H2}$ mittels des empirischen affinen Gesetzes zu schätzen, ferner dazu ausgestaltet ist, ein stöchiometrisches Luftvolumen oder einen Verbrennungsindex zu schätzen.

17. Vorrichtung nach einem der Ansprüche 12 bis 16, ferner umfassend ein Modul zur Regelung des Verbrennungsmerkmals des brennbaren Gases oder zur Regelung des Verbrennungsmerkmals eines brennbaren Gases und eines geschätzten stöchiometrischen Luftvolumens oder eines geschätzten Verbrennungsindexes, entsprechend dem geschätzten Merkmal.

**Claims**

1. A method for estimating at least one combustion characteristic of a fuel gas belonging to a family of fuel gases, said at least one characteristic being the Wobbe index or the higher heating value, the method comprising:

   a measurement (E01) of at least two flow properties of said fuel gas, the flow properties of the fuel gas being properties measured in a flow member and chosen from the group comprising the flow rate, the temperature, and the pressure,
   a measurement (E02) of the dihydrogen rate $X_{H_2}$ contained in said fuel gas,
   the method being **characterized in that**
   said at least one characteristic $\Xi_{GN/H2}$ is estimated (E03) by means of the following empirical affine law:

   $$\Xi_{GN/H2} = \alpha + \beta \cdot Y + \gamma \cdot X_{H_2}$$

   with $\alpha$, $\beta$ and $\gamma$ predetermined coefficients for the family of fuel gases, and
   Y a variable representative of physical properties of said fuel gas developed from said measured values of said at least two flow properties of said fuel gas, Y being a variable representative of physical properties of the fuel gas such as viscosity, specific heat capacity or density, and expressible as a function of the measured values of the flow properties of the fuel gas.

2. The method according to claim 1, wherein the coefficients $\alpha$, $\beta$ and $\gamma$ are coefficients read in a mapping having as input the measured value of the dihydrogen rate $X_{H_2}$ and outputting said coefficients $\alpha$, $\beta$ and $\gamma$.

3. The method according to claim 2, wherein said mapping associates said coefficients $\alpha$, $\beta$ and $\gamma$ with 1% wide value ranges of dihydrogen rate $X_{H2}$ .

4. The method according to any one of claims 1 to 3, wherein the values of said coefficients $\alpha$, $\beta$ and $\gamma$ are obtained from a set of data relating to known gases of said family of fuel gases for which the combustion characteristics and the value of Y representative of said physical properties are known.

5. The method according to claim 4, comprising a random generation of combustion characteristics and values of Y representative of physical properties from said set of data relating to known gases of said family of fuel gases.

6. The method according to any one of claims 1 to 5, the Wobbe index and the higher heating value are estimated by means of two empirical affine laws.

7. The method according to claim 6, further comprising an estimation of the density of said fuel gas from the estimated Wobbe index and the estimated higher heating value.

8. The method according to any one of claims 1 to 7, comprising a regulation of the combustion characteristic of the fuel gas or a regulation of the combustion characteristic of a fuel gas and an estimated stoichiometric air volume or an estimated combustibility index corresponding to said estimated characteristic.

9. The method according to any one of claims 1 to 8, wherein said at least one combustion characteristic of the fuel gas comprises the Wobbe index $IW_{GN/H2}$,

   said measurement of at least two flow properties of said fuel gas includes a measurement (E12) of a mass flow rate of the fuel gas in sonic flow through a fluidic restriction, performed at an absolute pressure measured upstream of the restriction and at an absolute temperature measured upstream of the restriction,
   the method further comprising a calibration procedure (E11) during which a measurement of a mass flow rate of a reference gas in sonic flow through said fluidic restriction is performed, at a measured reference absolute pressure and at a measured reference absolute temperature;
   the empirical affine law used to estimate (E14) the Wobbe index $IW_{GN/H2}$ being then written:

$$IW_{GN/H2} = D + E \cdot Y + F \cdot X_{H_2}$$

with:

$$Y = \frac{Q_{mes,2}}{Q_{ref}} \cdot \frac{p_{ref}}{p_{mes}} \cdot \sqrt{\frac{T_{ref}}{T_{mes}}}$$

where $Q_{mes,2}$ is the mass flow rate of the measured fuel gas,

$p_{mes}$ is the absolute pressure of the measured fuel gas,
$T_{mes}$ is the absolute temperature of the measured fuel gas,
$Q_{ref}$ is the mass flow rate of the measured reference gas,
$p_{ref}$ is the absolute pressure of the measured reference gas, and
$T_{ref}$ is the absolute temperature of the measured reference gas; and
D, E and F are predetermined coefficients for the family of fuel gases and corresponding respectively to the coefficients $\alpha$, $\beta$ and $\gamma$.

10. The method according to any one of claims 1 to 9, wherein the Wobbe index of said fuel gas has been estimated, the method further comprising a measurement of the density of said fuel gas and an estimation of the higher heating value from the estimated Wobbe index and the measured gas density.

11. The method according to any one of claims 1 to 9, wherein said at least one combustion characteristic of the fuel gas comprises the higher heating value $PCS_{GN/H2}$,
said measurement of at least two flow properties of said fuel gas includes:

a measurement (E22) of the mass flow rate of said fuel gas in laminar flow through an apparatus showing a pressure drop, the measurement depending on the viscosity of the fuel gas and on the viscosity of a reference gas, and
a measurement (E23), downstream of said apparatus showing a pressure drop, of the mass flow rate of said fuel gas by means of a thermal mass flow meter, the measurement depending on the specific heat capacity of the fuel gas and on the viscosity of a reference gas;
the empirical affine law used to estimate (E25) the higher heating value $PCS_{GN/H2}$ is then written

$$PCS_{GN/H2} = A + B \cdot Z + C \cdot X_{H_2}$$

with:

$$Z = \frac{Q_{mes,1}}{Q_{mes,2}}$$

where Z is a variable corresponding to the variable Y,

$Q_{mes,1}$ is the mass flow rate of the fuel gas in laminar flow through an apparatus showing a measured pressure drop, and
$Q_{mes,2}$ is the mass flow rate of the fuel gas measured downstream of said apparatus showing a pressure drop; and
A, B and C are predetermined coefficients for the family of fuel gases and corresponding respectively to the coefficients $\alpha$, $\beta$ and $\gamma$.

12. A device for estimating at least one combustion characteristic of a fuel gas belonging to a family of fuel gases, said at least one characteristic being the Wobbe index or the higher heating value, the device comprising:

at least two modules for measuring (3a, 3b) at least two flow properties of said fuel gas, the flow properties of the fuel gas being properties measured in a flow member and which are chosen from the group comprising the flow rate, the temperature and the pressure,
a module for measuring (4) the dihydrogen rate $X_{H2}$ contained in said fuel gas,
the device being **characterized by**
a module (5) configured to estimate said at least one characteristic $\Xi_{GN/H2}$ by means of the following empirical affine law:

$$\Xi_{GN/H2} = \alpha + \beta \cdot Y + \gamma \cdot X_{H_2}$$

with $\alpha$, $\beta$ and $\gamma$ predetermined coefficients for the family of fuel gases, and
Y a variable representative of physical properties of said fuel gas developed from said measured values of said at least two flow properties of said fuel gas, Y being a variable representative of physical properties of the fuel gas such as viscosity, specific heat capacity or density, and expressible as a function of the measured values of the flow properties of the fuel gas.

13. The device according to claim 12, wherein said at least one combustion characteristic of the fuel gas comprises the Wobbe index $IW_{GN/H2}$, the device comprising:

an inlet (11) for receiving a stream of said fuel gas,
an inlet (12) for receiving a stream of a reference gas,
a selection and guide module (31, 35) for bringing the stream of said fuel gas or the stream of said reference gas to a pipe (13),
a fluidic restriction (32),
a module for measuring (33) a mass flow rate of the fuel gas in sonic flow through said fluidic restriction, comprising a sub-module for measuring the absolute pressure upstream of the restriction and a sub-module for measuring the absolute temperature upstream of the restriction,
the empirical affine law used to estimate the Wobbe index $IW_{GN/H2}$ being then written:

$$IW_{GN/H2} = D + E \cdot Y + F \cdot X_{H_2}$$

with:

$$Y = \frac{Q_{mes,2}}{Q_{ref}} \cdot \frac{p_{ref}}{p_{mes}} \cdot \sqrt{\frac{T_{ref}}{T_{mes}}}$$

where $Q_{mes,2}$ is the mass flow rate of the measured fuel gas,

$p_{mes}$ is the absolute pressure of the measured fuel gas,
$T_{mes}$ is the absolute temperature of the measured fuel gas,
$Q_{ref}$ is the mass flow rate of the measured reference gas,
$p_{ref}$ is the absolute pressure of the measured reference gas, and
$T_{ref}$ is the absolute temperature of the measured reference gas; and
D, E and F are predetermined coefficients for the family of fuel gases and corresponding respectively to the coefficients $\alpha$, $\beta$ and $\gamma$.

14. The device according to claim 12 or 13, wherein the device is able to estimate the Wobbe index of said fuel gas, the device further comprising a module for measuring the density of said fuel gas and the module (5) configured to estimate said at least one characteristic being further configured to estimate the higher heating value from the estimated Wobbe index and the measured gas density.

15. The device according to claim 12 or 13, wherein said at least one combustion characteristic of the fuel gas comprises the higher heating value $PCS_{GN/H2}$, the device comprising:

an inlet (111) for receiving a stream of said fuel gas,

a module for measuring (132) the mass flow rate of said fuel gas in laminar flow through an apparatus showing a pressure drop, the measurement depending on the viscosity of the fuel gas and on the viscosity of a reference gas, and

a module for measuring (133), downstream of said apparatus showing a pressure drop, the mass flow rate of said fuel gas by means of a thermal mass flow meter, the measurement depending on the specific heat capacity of the fuel gas and on the heat capacity of a reference gas;

the empirical affine law used to estimate the higher heating value $PCS_{GN/H2}$ being then written

$$PCS_{GN/H2} = A + B \cdot Z + C \cdot X_{H_2}$$

with:

$$Z = \frac{Q_{mes,1}}{Q_{mes,2}}$$

where Z is a variable corresponding to the variable Y,

$Q_{mes,1}$ is the mass flow rate of the fuel gas in laminar flow through an apparatus showing a measured pressure drop, and

$Q_{mes,2}$ is the mass flow rate of the fuel gas measured downstream of said apparatus showing a pressure drop; and

*A, B* and *C* are predetermined coefficients for the family of fuel gases and corresponding respectively to the coefficients $\alpha$, $\beta$ and $\gamma$.

16. The device according to any of claims 12 to 15, wherein the module configured to estimate said at least one characteristic $\Xi_{GN/H2}$ by means of the empirical affine law is further configured to estimate a stoichiometric air volume or a combustibility index.

17. The device according to any of claims 12 to 16, further comprising a module for regulating said combustion characteristic of the fuel gas or for regulating the combustion characteristic of a fuel gas and an estimated stoichiometric air volume or an estimated combustibility index corresponding to said estimated characteristic.

E01 ⌐ Mesure de propriétés d'écoulement

E02 ⌐ Mesure du taux de dihydrogène

E03 ⌐ Estimation

# FIG.1

# FIG.2

E11 — | Etalonnage |

E12 — | Mesure de débit massique à pression absolue mesurée et température absolue mesurée |

E13 — | Mesure du taux de dihydrogène |

E14 — | Estimation |

## FIG.3

10

G1 →  11    31    13         32         33         34

G0 →  12    35

60  50

$Q_m$         $X\_H2$

p    T

36 —  51  52  41
         42
         43
54  53  44

37 —

## FIG.4

FIG.5

FIG.6

FIG.7

FIG.8

201

202

203

204

205

10

EP 3 563 152 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1346215 A
- US 4384792 A
- US 6244097 B
- DE 4118781
- US 5122746 A